# FASCICULE DE BREVET EUROPEEN

(11) **EP 1 142 556 B1**
(45) Date de publication et mention de la délivrance du brevet: **09.01.2008**
(21) Numéro de dépôt: 00403471.6
(22) Date de dépôt: 11.12.2000
(51) Int. Cl.: A61Q 5/10, A61K 8/34, A61K 8/81

(54) **Composition pour la teinture d'oxydation des fibres kératiniques comprenant un polymère épaississant comportant au moins une chaîne grasse et un alcool gras mono ou poly glycérolé**
Oxidationsfärbemittel für keratinische Fasern enthaltend ein verdickendes Polymer mit mindestens einer hydrophoben Seitenkette und einen Mono- oder Polyglycerol- Fettalkohol
Composition for oxidative dyeing of keratinous fibres containing a thickener polymer with a fatty chain and a mono- or polyglycerolated fatty alcohol

(30) Priorité: 30.12.1999 FR 9916757
(43) Date de publication de la demande: 10.10.2001
(73) Titulaire: L'ORÉAL, 75008 Paris (FR)
(72) Inventeur: Cottard, François, 92300 Levallois Perret (FR); Rondeau, Christine, 78500 Sartrouville (FR)
(74) Mandataire: Miszputen, Laurent

(56) Documents cités:
- EP-A- 0 384 415
- EP-A- 0 490 053
- EP-A- 0 557 203
- DE-A- 19 714 370
- DE-A- 19 757 509
- FR-A- 2 428 437
- FR-A- 2 773 472
- FR-A- 2 773 475

## Description

La présente invention concerne une composition pour la teinture d'oxydation des fibres kératiniques, en particulier des fibres kératiniques humaines telles que les cheveux, comprenant, dans un milieu approprié pour la teinture, au moins un colorant d'oxydation, au moins un polymère épaississant comportant au moins une chaîne grasse et au moins un alcool gras mono- ou poly-glycérolé.

Il est connu de teindre les fibres kératiniques et en particulier les cheveux humains, avec des compositions de teinture contenant des précurseurs de colorants d'oxydation, généralement appelés "bases d'oxydation", en particulier des ortho- ou para- phénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des "bases d'oxydation" sur elles-mêmes, soit d'une condensation oxydative des "bases d'oxydation" sur des composés modificateurs de coloration, ou "coupleurs", qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les "bases d'oxydation" et d'autre part par les "coupleurs", permet l'obtention d'une palette très riche en coloris.

Pour localiser le produit de coloration d'oxydation à l'application sur les cheveux afin qu'il ne coule pas sur le visage ou en dehors des zones que l'on se propose de teindre, on a jusqu'ici eu recours à l'emploi d'épaississants traditionnels tels que l'acide polyacrylique réticulé, les hydroxyéthylcelluloses, certains polyuréthanes, les cires ou encore à des mélanges d'agents tensio-actifs non-ioniques de HLB (Hydrophilic Lipophilic Balance), qui, convenablement choisis, engendrent l'effet gélifiant quand on les dilue au moyen d'eau et/ou d'agents tensio-actifs.

Cependant, la demanderesse a constaté que les systèmes épaississants mentionnés ci-dessus ne permettaient pas d'obtenir des nuances puissantes et chromatiques de faibles sélectivités et de bonnes ténacités tout en assurant un bon état cosmétique à la chevelure traitée. Par ailleurs, elle a également constaté que les compositions tinctoriales prêtes à l'emploi contenant le ou les colorants d'oxydation, et les systèmes épaississants de l'art antérieur ne permettaient pas une application suffisamment précise sans coulures ni chutes de viscosité dans le temps.

La demande EP 557203 a pour objet des compositions utilisées notamment dans le traitement des fibres kératiniques humaines, comme la coloration, la décoloration ou la déformation permanente. Ces compositions comprennent un mélange de tensioactifs non ioniques polyoxyéthylénés et/ou polyoxypropylénés et/ou polyglycérolés de HLB particulières, associé à un polymère substantif cationique ou amphotère, notamment dans le but de résoudre des problèmes d'incompatibilité entre les tensioactifs non ioniques et des polymères substantifs cationiques.

On connaît avec la demande FR 2773472 des compositions tinctoriales comprenant une enzyme de type laccase, au moins un précurseur de colorant d'oxydation, au moins un polymère épaississant dont une liste particulière est donnée, parmi lesquels figurent des polymères anioniques ou non ioniques, porteurs ou non d'une chaîne grasse. Ces compositions permettent d'améliorer les propriétés tinctoriales de compositions.

Or, après d'importantes recherches menées sur la question, la Demanderesse vient maintenant de découvrir qu'il est possible d'obtenir des compositions de teinture d'oxydation prêtes à l'emploi qui ne coulent pas et restent donc bien localisées au point d'application, et qui permettent aussi d'obtenir des nuances puissantes et chromatiques (lumineuses) avec de faibles sélectivités et de bonnes ténacités vis à vis des agents chimiques ( shampooing, permanentes ...) ou naturels (lumière, transpiration ...) tout en apportant aux cheveux de bonnes propriétés cosmétiques en ajoutant à la composition tinctoriale au moins un polymère épaississant comportant au moins une chaine grasse, et au moins un alcool gras mono- ou poly-glycérolé.

La Demanderesse a également découvert que les compositions contenant au moins un colorant d'oxydation, au moins un polymère épaississant comportant au moins une chaîne une chaîne grasse et au moins un alcool gras mono- ou poly-glycérolé présentaient une très bonne stabilité au stockage.

Ces découvertes sont à la base de la présente invention.

La présente invention a ainsi pour objet une composition pour la teinture d'oxydation des fibres kératiniques, en particulier des fibres kératiniques humaines, telles que les cheveux, comprenant, dans un milieu approprié pour la teinture, au moins un colorant d'oxydation, et caractérisée par le fait qu'elle comprend en outre au moins un polymère épaississant comportant au moins une chaîne grasse et au moins un alcool gras mono- ou poly-glycérolé.

Un autre objet de l'invention porte sur une composition prête à l'emploi pour la teinture d'oxydation des fibres kératiniques qui comprend, dans un milieu approprié pour la teinture, au moins un colorant d'oxydation, au moins un polymère épaississant comportant au moins une chaîne grasse, au moins un alcool gras mono- ou poly-glycérolé, et au moins un agent oxydant. Par composition prête à l'emploi, on entend au sens de la présente invention, la composition destinée à être appliquée immédiatement sur les fibres kératiniques, c'est à dire qu'elle peut être stockée telle quelle avant utilisation ou résulter du mélange extemporané de deux ou plusieurs compositions.

L'invention vise également un procédé de teinture d'oxydation des fibres kératiniques, et en particulier des fibres kératiniques humaines telles que les cheveux, consistant à appliquer sur les fibres une composition (A) contenant, dans un milieu approprié pour la teinture, au moins un colorant d'oxydation en association avec au moins un polymère épaississant comportant au moins une chaîne grasse et au moins un alcool gras mono- ou poly-glycérolé, la couleur étant révélée à pH alcalin, neutre ou acide à l'aide d'une composition (B) contenant au moins un agent oxydant, qui est mélangée juste au moment de l'emploi à la composition (A) ou qui est appliquée séquentiellement sans rinçage intermédiaire.

L'invention vise aussi une variante de ce procédé, qui consiste à appliquer sur les fibres une composition colorante contenant dans un milieu approprié pour la teinture, au moins un colorant d'oxydation et au moins un alcool gras mono- ou poly-glycérolé, et ceci en la présence ou l'absence d'au moins un polymère épaississant comportant au moins une chaîne grasse, la couleur étant révélée à pH alcalin, neutre ou acide à l'aide d'une composition oxydante qui contient un agent oxydant et une quantité efficace d'au moins un polymère épaississant comportant au moins une chaîne grasse et qui est mélangée juste au moment de l'emploi à la composition colorante ou qui est appliquée séquentiellement sans rinçage intermédiaire.

L'invention a également pour objet des dispositifs de teinture à plusieurs compartiments ou " kits " pour la teinture d'oxydation des fibres kératiniques, en particulier des fibres kératiniques humaines telles que les cheveux.
Un dispositif selon l'invention comporte un premier compartiment contenant au moins un colorant d'oxydation et au moins un polymère épaississant comportant au moins une chaîne grasse et au moins un alcool gras mono- ou poly-glycérolé, et un deuxième compartiment contenant un agent oxydant.
Selon une variante, un autre dispositif comporte un premier compartiment contenant au moins un colorant d'oxydation et au moins un alcool gras mono- ou poly-glycérolé, et ceci en la présence ou l'absence d'au moins un polymère épaississant comportant au moins une chaîne grasse et un deuxième compartiment contenant un agent oxydant et une quantité efficace d'au moins un polymère épaississant comportant au moins une chaîne grasse.
Selon une autre variante, un dispositif à trois compartiments comporte un premier compartiment contenant au moins un colorant d'oxydation et au moins un alcool gras mono- ou poly-glycérolé, et ceci en la présence ou l'absence d'au moins un polymère épaississant comportant au moins une chaîne grasse, un second compartiment contenant au moins un polymère épaississant comportant au moins une chaîne grasse et un troisième compartiment contenant un agent oxydant en la présence ou en l'absence de polymère épaississant comportant au moins une chaîne grasse.

Mais d'autres caractéristiques, aspects, objets et avantages de l'invention apparaîtront encore plus clairement à la lecture de la description et des exemples qui suivent.

Par alcool gras mono- ou poly- glycérolé, on entend tout composé présentant la structure suivante :

RO- ( -CH₂-CH(CH₂OH)-O -) ₙ-H

dans laquelle :
R représente un radical saturé ou insaturé, linéaire ou ramifié, comportant de 8 à 40 atomes de carbone et de préférence de 10 à 30 atomes de carbone ; n représente un nombre compris entre 1 et 30 et de préférence entre 1 et 10.

Comme composés de ce type on peut citer l'alcool laurique à 4 moles de glycérol (nom INCl : POLYGLYCERYL-4 LAURYL ETHER), l'alcool oléique à **4** moles de glycérol (nom INCl : POLYGLYCERYL-4 OLEYL ETHER), l'alcool oléique à 2 moles de glycérol (Nom INCl : POLYGLYCERYL-2 OLEYL ETHER), l'alcool cétéarylique à 2 moles de glycérol, l'alcool cétéarylique à 6 moles de glycérol , l'alcool oléocétylique à 6 moles de glycérol, et l'octadécanol à 6 moles de glycérol.
L'alcool gras peut représenter un mélange d'alcools gras au même titre que la valeur de n représente une valeur statistique, ce qui signifie que dans un produit commercial peuvent coexister plusieurs espèces d'alcools gras polyglycérolés sous forme d'un mélange.
Les alcools gras mono- ou poly- glycérolés représentent environ de 0,01 à 30%, de préférence environ de 0,05 à 20% et plus préférentiellement environ de 0,1 à 15% du poids total de la composition.

Les polymères épaississants comportant au moins une chaîne grasse selon l'invention, sont de préférence de type non ionique, anionique, ou cationique.

Parmi les polymères épaississants comportant au moins une chaîne grasse et de type anionique, on peut citer:
-(**I**) ceux comportant au moins un motif hydrophile, et au moins un motif éther d'allyle à chaîne grasse, plus particulièrement ceux dont le motif hydrophile est constitué par un monomère anionique insaturé éthylénique, plus particulièrement encore par un acide carboxylique vinylique et tout particulièrement par un acide acrylique ou un acide méthacrylique ou les mélanges de ceux ci, et dont le motif éther d'allyle à chaîne grasse correspond au monomère de formule (I) suivante :

   CH₂ = C R' CH₂ O Bₙ R (I)

   dans laquelle R' désigne H ou CH₃, B désigne le radical éthylèneoxy, n est nul ou désigne un entier allant de 1 à 100, R désigne un radical hydrocarboné choisi parmi les radicaux alkyl, arylalkyle, aryle, alkylaryle, cycloalkyle, comprenant de 8 à 30 atomes de carbone, de préférence 10 à 24, et plus particulièrement encore de 12 à 18 atomes de carbone. Un motif de formule (I) plus particulièrement préféré est un motif dans lequel R' désigne H, n est égal à 10, et R désigne un radical stéaryl (C₁₈).
   Des polymères amphiphiles anioniques de ce type sont décrits et préparés, selon un procédé de polymérisation en émulsion, dans le brevet EP-0 216 479.
   Parmi ces polymères épaississants anioniques à chaîne grasse, on préfère particulièrement selon l'invention, les polymères formés à partir de 20 à 60% en poids d'acide acrylique et/ou d'acide méthacrylique, de 5 à 60% en poids de (méth)acrylates d'alkyles inférieurs, de 2 à 50% en poids d'éther d'allyl à chaîne grasse de formule (I), et de 0 à 1% en poids d'un agent réticulant qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
   Parmi ces derniers, on préfère tout particulièrement les terpolymères réticulés d'acide méthacrylique, d'acrylate d'éthyle, de polyéthylèneglycol (10 OE) éther d'alcool stéarylique (Steareth 10), notamment ceux vendus par la société ALLIED COLLOIDS sous les dénominations SALCARE SC 80 et SALCARE SC90 qui sont des émulsions aqueuses à 30% d'un terpolymère réticulé d'acide méthacrylique, d'acrylate d'éthyle et de steareth-10-allyl éther (40/50/10).
-(**II**) ceux comportant au moins un motif hydrophile de type acide carboxylique insaturé oléfinique, et au moins un motif hydrophobe de type ester d'alkyl (C₁₀-C₃₀) d'acide carboxylique insaturé.
   De préférence, ces polymères sont choisis parmi ceux dont le motif hydrophile de type acide carboxylique insaturé oléfinique correspond au monomère de formule (II) suivante : 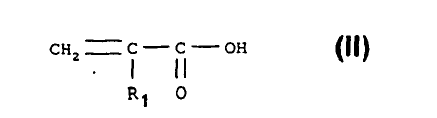 dans laquelle, R₁ désigne H ou CH₃ ou C₂H₅, c'est-à-dire des motifs acide acrylique, acide méthacrylique ou acide éthacrylique, et dont le motif hydrophobe de type ester d'alkyl (C₁₀-C₃₀) d'acide carboxylique insaturé correspond au monomère de formule (III) suivante : 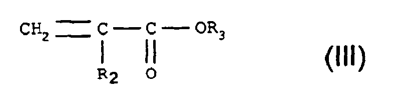 dans laquelle, R₂ désigne H ou CH₃ ou C₂H₅ (c'est-à-dire des motifs acrylates, méthacrylates ou éthacrylates) et de préférence H (motifs acrylates) ou CH₃ (motifs méthacrylates), R₃ désignant un radical alkyle en C₁₀-C₃₀, et de préférence en C₁₂-C₂₂.
   Des esters d'alkyles (C₁₀-C₃₀) d'acides carboxyliques insaturés conformes à l'invention comprennent par exemple, l'acrylate de lauryle, l'acrylate de stéaryle, l'acrylate de décyle, l'acrylate d'isodécyle, l'acrylate de dodécyle, et les méthacrylates correspondants, le méthacrylate de lauryle, le méthacrylate de stéaryle, le méthacrylate de décyle, le méthacrylate d'isodécyle, et le méthacrylate de dodécyle.
   Des polymères anioniques de ce type sont par exemple décrits et préparés, selon les brevets US-3 915 921 et 4 509 949.
   Parmi ce type de polymères épaississants anioniques à chaîne grasse, on utilisera plus particulièrement des polymères formés à partir d'un mélange de monomères comprenant :
   (i) essentiellement de l'acide acrylique,
   (ii) un ester de formule (III) décrite ci-dessus et dans laquelle R₂ désigne H ou CH₃, R₃ désignant un radical alkyle ayant de 12 à 22 atomes de carbone,
   (iii) et un agent réticulant, qui est un monomère insaturé polyéthylénique copolymérisable bien connu, comme le phtalate de diallyle, le (méth)acrylate d'allyl, le divinylbenzène, le diméthacrylate de (poly)éthylèneglycol, et le méthylène-bis-acrylamide.
   Parmi ce type de polymères épaississants anioniques à chaîne grasse, on utilisera plus particulièrement ceux constitués. de 95 à 60% en poids d'acide acrylique (motif hydrophile), 4 à 40% en poids d'acrylate d'alkyles en C₁₀-C₃₀ (motif hydrophobe), et 0 à 6% en poids de monomère polymérisable réticulant, ou bien ceux constitués de 98 à 96% en poids d'acide acrylique (motif hydrophile), 1 à 4% en poids d'acrylate d'alkyles en C₁₀-C₃₀ (motif hydrophobe), et 0,1 à 0,6% en poids de monomère polymérisable réticulant tel que ceux décrits précedemment. Parmi lesdits polymères ci-dessus, on préfère tout particulièrement selon la présente invention, les produits vendus par la société GOODRICH sous les dénominations commerciales PEMULEN TR1, PEMULEN TR2, CARBOPOL 1382, et encore plus préférentiellement le PEMULEN TR1, et le produit vendu par la société S.E.P.P.I.C. sous la dénomination COATEX SX.
-(**III**) les terpolymères d'anhydride maléique/α-oléfine en C₃₀-C₃₈/ maléate d'alkyle tel que le produit (copolymère anhydride maléique/α-oléfine en C₃₀-C₃₈/maléate d'isopropyle) vendu sous le nom PERFORMA V 1608 par la société NEWPHASE TECHNOLOGIES.
-(**IV**) les terpolymères acryliques comprenant :
   (a) environ 20% à 70% en poids d'un acide carboxylique à insaturation α,β-monoéthylénique,
   (b) environ 20 à 80% en poids d'un monomère à insaturation α,β-monoéthylénique non-tensio-actif différent de (a),
   (c) environ 0,5 à 60% en poids d'un mono-uréthane non-ionique qui est le produit de réaction d'un tensio-actif monohydrique avec un monoisocyanate à insaturation monoéthylénique,
   tels que ceux décrits dans la demande de brevet EP-A-0173109 et plus particulièrement celui décrit dans l'exemple 3, à savoir, un terpolymère acide méthacrylique /acrylate de méthyle/diméthyl métaisopropényl benzyl isocyanate d'alcool béhényle éthoxylé (400E) en dispersion aqueuse à 25%.
-(**V**) les copolymères comportant parmi leurs monomères un acide carboxylique à insaturation α,β-monoéthylénique et un ester d'acide carboxylique à insaturation α,β-monoéthylénique et d'un alcool gras oxyalkyléné.

Préférentiellement ces composés comprennent également comme monomère un ester d'acide carboxylique à insaturation α,β-monoéthylénique et d'alcool en C1C4.
A titre d'exemple de ce type de composé on peut citer l'ACULYN 22 vendu par la société ROHM et HAAS, qui est un terpolymère acide méthacrylique/acrylate d'éthyle/méthacrylate de stéaryle oxyalkyléné.

Les polymères épaississants à chaîne grasse de type non ionique, utilisés selon l'invention, sont choisis de préférence parmi :
-(**1**) les celluloses modifiées par des groupements comportant au moins une chaîne grasse ;
   on peut citer à titre d'exemple :
   - les hydroxyéthylcelluloses modifiées par des groupements comportant au moins une chaîne grasse tels que des groupes alkyle, arylalkyle, alkylaryle, ou leurs mélanges, et dans lesquels les groupes alkyle sont de préférence en C₈-C₂₂, comme le produit NATROSOL PLUS GRADE 330 CS (alkyles en C₁₆) vendu par la société AQUALON, ou le produit BERMOCOLL EHM 100 vendu par la société BEROL NOBEL,
   - celles modifiées par des groupes polyalkylène glycol éther d'alkyl phénol, tel que le produit AMERCELL POLYMER HM-1500 (polyéthylène glycol (15) éther de nonyl phénol) vendu par la société AMERCHOL.
-(**2**) les hydroxypropylguars modifiés par des groupements comportant au moins une chaîne grasse tel que le produit ESAFLOR HM 22 (chaîne alkyle en C₂₂) vendu par la société LAMBERTI, les produits RE210-18 (chaîne alkyle en C₁₄) et RE205-1 (chaîne alkyle en C₂₀) vendus par la société RHONE POULENC.
-(**3**) les copolymères de vinyl pyrrolidone et de monomères hydrophobes à chaîne grasse ;
   on peut citer à titre d'exemple :
   - les produits ANTARON V216 ou GANEX V216 (copolymère vinylpyrrolidone / hexadécène) vendu par la société I.S.P.
   - les produits ANTARON V220 ou GANEX V220 (copolymère vinylpyrrolidone / eicosène) vendu par la société I.S.P.
   - (**4**) les copolymères de méthacrylates ou d'acrylates d'alkyles en C₁-C₆ et de monomères amphiphiles comportant au moins une chaîne grasse tels que par exemple le copolymère acrylate de méthyle/acrylate de stéaryle oxyéthyléné vendu par la société GOLDSCHMIDT sous la dénomination ANTIL 208.
   - (**5**) les copolymères de méthacrylates ou d'acrylates hydrophiles et de monomères hydrophobes comportant au moins une chaîne grasse tels que par exemple le copolymère méthacrylate de polyéthylèneglycol/méthacrylate de lauryle.
   - (**6**) les polyuréthanes polyéthers comportant dans leur chaîne, à la fois des séquences hydrophiles de nature le plus souvent polyoxyéthylénée et des séquences hydrophobes qui peuvent être des enchaînements aliphatiques seuls et/ou des enchaînements cycloaliphatiques et/ou aromatiques.
   - (**7**) les polymères à squelette aminoplaste éther possédant au moins une chaîne grasse, tels que les composés PURE THIX proposés par la société SUD-CHEMIE.

De préférence, les polyéthers polyuréthanes comportent au moins deux chaînes lipophiles hydrocarbonées, ayant de 6 à 30 atomes de carbone, séparées par une séquence hydrophile, les chaînes hydrocarbonées pouvant être des chaînes pendantes ou des chaînes en bout de séquence hydrophile. En particulier, il est possible qu'une ou plusieurs chaînes pendantes soient prévues. En outre, le polymère peut comporter, une chaîne hydrocarbonée à un bout ou aux deux bouts d'une séquence hydrophile.
Les polyéthers polyuréthanes peuvent être multiséquencés en particulier sous forme de tribloc. Les séquences hydrophobes peuvent être à chaque extrémité de la chaîne (par exemple : copolymère tribloc à séquence centrale hydrophile) ou réparties à la fois aux extrémités et dans la chaîne (copolymère multiséquencé par exemple). Ces mêmes polymères peuvent être également en greffons ou en étoile.
Les polyéthers polyuréthanes non-ioniques à chaîne grasse peuvent être des copolymères triblocs dont la séquence hydrophile est une chaîne polyoxyéthylénée comportant de 50 à 1000 groupements oxyéthylénés. Les polyéthers polyuréthanes non-ioniques comportent une liaison uréthanne entre les séquences hydrophiles, d'où l'origine du nom.
Par extension figurent aussi parmi les polyéthers polyuréthanes non-ioniques à chaîne grasse, ceux dont les séquences hydrophiles sont liées aux séquences lipophiles par d'autres liaisons chimiques.
A titre d'exemples de polyéthers polyuréthanes non-ioniques à chaîne grasse utilisables dans l'invention, on peut aussi utiliser aussi le Rhéolate 205 à fonction urée vendu par la société RHEOX ou encore les Rhéolates 208 , 204 ou 212, ainsi que l'Acrysol RM 184, l'Aculyn 44 et l'Aculyn 46 de la société ROHM & HAAS [l'ACULYN 46 est un polycondensat de polyéthylèneglycol à 150 ou 180 moles d'oxyde d'éthylène, d'alcool stéarylique et de méthylène bis(4-cyclohexyl-isocyanate) (SMDI), à 15% en poids dans une matrice de maltodextrine (4%) et d'eau (81%); l'ACULYN 44 est un polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%)].

On peut également citer le produit ELFACOS T210 à chaîne alkyle en C₁₂₋₁₄ et le produit ELFACOS T212 à chaîne alkyle en C₁₈ de chez AKZO.
Le produit DW 1206B de chez ROHM & HAAS à chaîne alkyle en C₂₀ et à liaison uréthanne, proposé à 20 % en matière sèche dans l'eau, peut aussi être utilisé.
On peut aussi utiliser des solutions ou dispersions de ces polymères notamment dans l'eau ou en milieu hydroalcoolique. A titre d'exemple, de tels polymères on peut citer, le Rhéolate 255, le Rhéolate 278 et le Rhéolate 244 vendus par la société RHEOX. On peut aussi utiliser le produit DW 1206F et le DW 1206J proposés par la société ROHM & HAAS.
Les polyéthers polyuréthanes utilisables selon l'invention sont en particulier ceux décrits dans l'article de G. Fonnum, J. Bakke et Fk. Hansen - Colloid Polym. Sci 271, 380.389 (1993).

Les polymères épaississants à chaîne grasse de type cationique utilisés dans la présente invention sont choisis de préférence parmi les dérivés de cellulose quaternisée et les polyacrylates à groupements latéraux aminés non cycliques.

Les dérivés de cellulose quaternisée sont, en particulier,
- les celluloses quaternisées modifiées par des groupements comportant au moins une chaîne grasse, tels que les groupes alkyle, arylalkyle, alkylaryle comportant au moins 8 atomes de carbone, ou des mélanges de ceux-ci,
- les hydroxyéthylcelluloses quaternisées modifiées par des groupements comportant au moins une chaîne grasse, tels que les groupes alkyle, arylalkyle, alkylaryle comportant au moins 8 atomes de carbone, ou des mélanges de ceux-ci.

Les radicaux alkyle portés par les celluloses ou hydroxyéthylcelluloses quaternisées ci-dessus comportent de préférence de 8 à 30 atomes de carbone. Les radicaux aryle désignent de préférence les groupements phényle, benzyle, naphtyle ou anthryle.
On peut indiquer comme exemples d'alkylhydroxyéthyl-celluloses quaternisées à chaînes grasses en C₈-C₃₀, les produits QUATRISOFT LM 200, QUATRISOFT LM-X 529-18-A, QUATRISOFT LM-X 529-18B (alkyle en C₁₂) et QUATRISOFT LM-X 529-8 (alkyle en C₁₈) commercialisés par la société AMERCHOL et les produits CRODACEL QM, CRODACEL QL (alkyle en C₁₂) et CRODACEL QS (alkyle en C18) commercialisés par la société CRODA.

Les polyacrylates à groupements latéraux aminés, quaternisés ou non, possèdent par exemple des groupements hydrophobes du type stéareth 20 (alcool stéarylique polyoxyéthyléné(20)).
Comme exemples de polyacrylates à chaînes latérales aminées, on peut citer les polymères 8781- 121B ou 9492-103 proposés par la société NATIONAL STARCH.

Dans la composition de teinture d'oxydation selon l'invention, on préfère utiliser un polymère épaississant à chaîne grasse de type non ionique.

Les polymères épaississants à chaîne grasse de type anionique, non ionique ou cationique sont utilisés de préférence en une quantité pouvant varier d'environ 0,01 à 10% en poids du poids total de la composition de teinture. Plus préférentiellement, cette quantité varie d'environ 0,1 à 5% en poids.

Les colorants d'oxydation utilisables selon l'invention sont choisis parmi les bases d'oxydation et/ou les coupleurs.
De préférence les compositions selon l'invention contiennent au moins une base d'oxydation.
Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para- aminophénols, les bases hétérocycliques suivantes ainsi que leurs sels d'addition avec un acide.
On peut notamment citer :
- (**I**) les paraphénylènediamines de formule (I) suivante et leurs sels d'addition avec un acide : 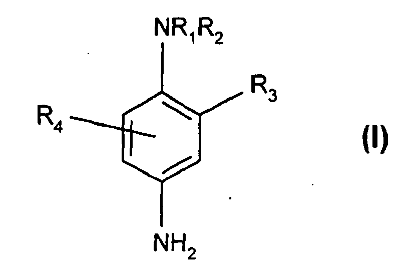 dans laquelle :
   R₁ représente un atome d'hydrogène, un radical alkyle en C₁-C₄, monohydroxyalkyle en C₁-C₄, polyhydroxyalkyle en C₂-C₄ , alcoxy(C₁-C₄)alkyle(C₁-C₄), alkyle en C₁-C₄ substitué par un groupement azoté, phényle ou 4'-aminophényle ;
   R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
   R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
   R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
   R₄ représente un atome d'hydrogène, d'halogène ou un radical alkyle en C₁-C₄.

Parmi les groupements azotés de la formule (I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les paraphénylènediamines de formule (I) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènédiamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylénediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylènediamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
-(**II)** Selon l'invention, on entend par bases doubles, les composés comportant au moins deux noyaux aromatiques sur lesquels sont portés des groupements amino et/ou hydroxyle.

Parmi les bases doubles utilisables à titre de bases d'oxydation dans les compositions tinctoriales conformes à l'invention, on peut notamment citer les composés répondant à la formule (II) suivante, et leurs sels d'addition avec un acide : 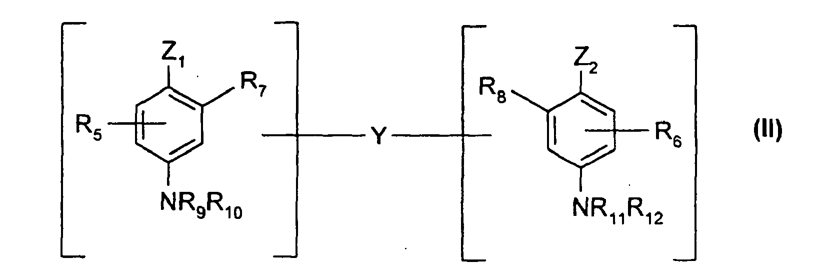 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

Parmi les groupements azotés de la formule (**I**I) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formules (II) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylënediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.

Parmi ces bases doubles de formule (II), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.
- (**III**) les para-aminophénols répondant à la formule (III) suivante, et leurs sels d'addition avec un acide : 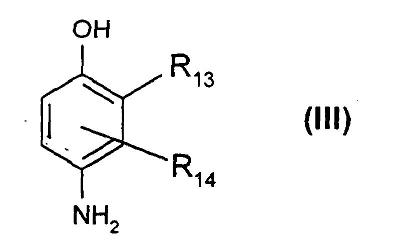 dans laquelle :
   R₁₃ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

Parmi les para-aminophénols de formule (III) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
- (**IV**) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
-(**V**) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diarnino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Selon la présente invention, les bases d'oxydation représentent de préférence de 0,0005 à 12% en poids environ du poids total de la composition et encore plus préférentiellement de 0,005 à 8% en poids environ de ce poids.

Les coupleurs utilisables dans la composition de teinture selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1 -(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthytamino)-1-méthoxy-benzéne, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Lorsqu'ils sont présents, ces coupleurs représentent de préférence de 0,0001 à 10% en poids environ du poids total de la composition, et encore plus préférentiellement de 0,005 à 5% en poids environ.

D'une manière générale, les sels d'addition avec un acide des bases d'oxydation et coupleurs sont notamment choisis parmi les chlorhydrates, les bromhydrates, les sulfates et les tartrates, les lactates et les acétates.

La composition selon l'invention peut encore contenir, en plus des colorants d'oxydation définis ci-dessus, des colorants directs pour enrichir les nuances en reflets. Ces colorants directs peuvent notamment alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

Dans la composition prête à l'emploi selon l'invention, la composition (A) et/ou la composition (B) peut en outre plus particulièrement contenir, au moins un polymère substantif cationique ou amphotère.

### Polymères cationiques

Au sens de la présente invention, l'expression "polymère cationique" désigne tout polymère contenant des groupements cationiques et/ou des groupements ionisables en groupements cationiques.

Les polymères cationiques utilisables conformément à la présente invention peuvent être choisis parmi tous ceux déja connus en soi comme améliorant les propriétés cosmétiques des cheveux, à savoir notamment ceux décrits dans la demande de brevet EP-A-337 354 et dans les brevets français FR-2 270 846, 2 383 660, 2 598 611, 2 470 596 et 2 519 863.

Les polymères cationiques préférés sont choisis parmi ceux qui contiennent des motifs comportant des groupements amine primaire, secondaire, tertiaire et/ou quaternaire pouvant, soit faire partie de la chaîne principale polymère, soit être portés par un substituant latéral directement relié à celle-ci.

Les polymères cationiques utilisés ont généralement une masse moléculaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus. Ils sont notamment décrits dans les brevets français n° 2 505 348 ou 2 542 997. Parmi lesdits polymères, on peut citer :
(**1**) Les homopolymères ou copolymères dérivés d'esters ou d'amides acryliques ou méthacryliques et comportant au moins un des motifs de formules (I), (II), (III) ou (IV) suivantes: 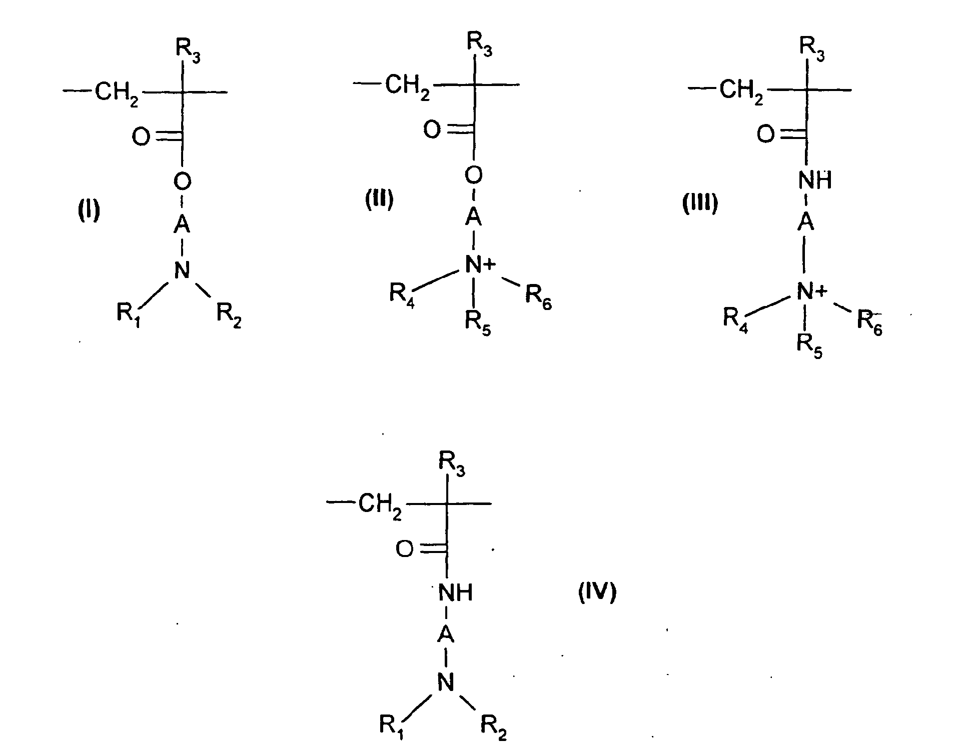 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂, identiques ou différents, représentent hydrogène ou un groupe alkyle ayant de 1 à 6 atomes de carbone et de préférence méthyle ou éthyle;
   X désigne un anion dérivé d'un acide minéral ou organique tel qu'un anion méthosulfate ou un halogénure tel que chlorure ou bromure.

Les polymères de la famille (**1**) peuvent contenir en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C₁-C₄), des acides acryliques ou méthacryliques ou leurs esters, des vinyllactames tels que la vinylpyrrolidone ou le vinylcaprolactame, des esters vinyliques.
Ainsi, parmi ces polymères de la famille (1), on peut citer :
- les copolymères d'acrylamide et de diméthylaminoéthyl méthacrylate quaternisé au sulfate de diméthyle ou avec un hologénure de diméthyle, tel que celui vendu sous la dénomination HERCOFLOC par la société HERCULES,
- les copolymères d'acrylamide et de chlorure de méthacryloyloxyéthyltriméthyl-ammonium décrits par exemple dans la demande de brevet EP-A-080976 et vendus sous la dénomination BINA QUAT P 100 par la société CIBA GEIGY,
- le copolymère d'acrylamide et de méthosulfate de méthacryloyloxyéthyltriméthyl-ammonium vendu sous la dénomination RETEN par la société HERCULES,
- les copolymères vinylpyrrolidone / acrylate ou méthacrylate de dialkylaminoalkyle quaternisés ou non, tels que les produits vendus sous la dénomination "GAFQUAT" par la société ISP comme par exemple "GAFQUAT 734" ou "GAFQUAT 755" ou bien les produits dénommés "COPOLYMER 845, 958 et 937". Ces polymères sont décrits en détail dans les brevets français 2.077.143 et 2.393.573,
- les terpolymères méthacrylate de diméthyl amino éthyle/ vinylcaprolactame/ vinylpyrrolidone tel que le produit vendu sous la dénomination GAFFIX VC 713 par la société ISP,
- les copolymère vinylpyrrolidone / méthacrylamidopropyl dimethylamine commercialisés notamment sous la dénomination STYLEZE CC 10 par ISP,
- et les copolymères vinylpyrrolidone / méthacrylamide de diméthylaminopropyle quaternisés tel que le produit vendu sous la dénomination "GAFQUAT HS 100" par la société ISP.

(**2**) Les dérivés d'éthers de cellulose comportant des groupements ammonium quaternaire décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium .
(**3**) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyldiallylammonium.
   Les produits commercialisés répondant à cette définition sont plus particulièrement les produits vendus sous la dénomination "Celquat L 200" et "Celquat H 100" par la Société National Starch.
(**4**) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(**5**) Les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361.
(**6**) Les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polyaminoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 .
(**7**) Les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(**8**) Les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d'épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(**9**) Les cyclopolymères d'alkyl diallyl amine ou de dialkyl diallyl ammonium tels que les homopolymères ou copolymères comportant comme constituant principal de la chaîne des motifs répondant aux formules (V) ou (VI) : 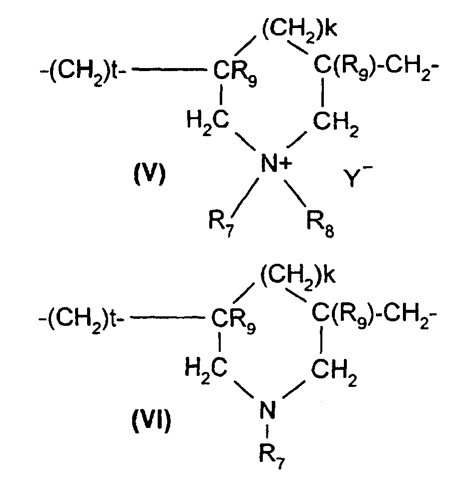 formules dans lesquelles k et t sont égaux à 0 ou 1, la somme k + t étant égale à 1 ; R₉ désigne un atome d'hydrogène ou un radical méthyle ; R₇ et R₈, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C₁-C₄), ou R₇ et R₈ peuvent désigner conjointement avec l'atome d'azote auquel ils sont rattachés, des groupement hétérocycliques, tels que pipéridinyle ou morpholinyle ; R₇ et R₈ indépendamment l'un de l'autre désignent de préférence un groupement alkyle ayant de 1 à 4 atomes de carbone ; Y- est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Calgon (et ses homologues de faible masse moléculaire moyenne en poids) et les copolymères de chlorure de diallyldiméthylammonium et d'acrylamide commercialisés sous la dénomination "MERQUAT 550".
(**10**) Le polymère de diammonium quaternaire contenant des motifs récurrents répondant à la formule : 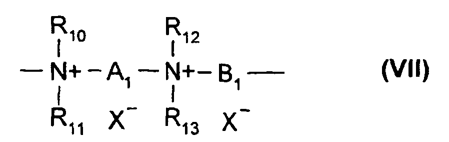 formule (VII) dans laquelle :
   R₁₀, R₁₁, R₁₂ et R₁₃, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₀, R₁₁,
   R₁₂ et R₁₃, ensemble ou séparément, constituent avec les atomes d'azote auxquels ils sont rattachés des hétérocycles contenant éventuellement un second hétéroatome autre que l'azote ou bien R₁₀, R₁₁, R₁₂ et R₁₃ représentent un radical alkyle en C₁-C₆ linéaire ou ramifié substitué par un groupement nitrile, ester, acyle, amide ou -CO-O- R₁₄-D ou -CO-NH- R₁₄-D où R₁₄ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R₁₀ et R₁₂ peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement -(CH₂)n-CO-D-OC-(CH₂)n- dans lequel D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- .

De préférence, X⁻ est un anion tel que le chlorure ou le bromure.
Ces polymères ont une masse moléculaire moyenne en nombre généralement comprise entre 1000 et 100000.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule (VIII) suivante: 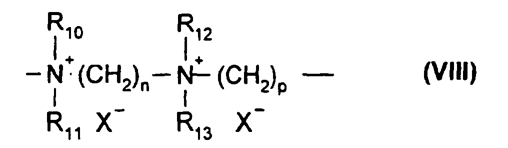 dans laquelle R₁₀, R₁₁, R₁₂ et R₁₃, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.
**(11)** Les polymères de polyammonium quaternaire constitués de motifs récurrents de formule (IX) : 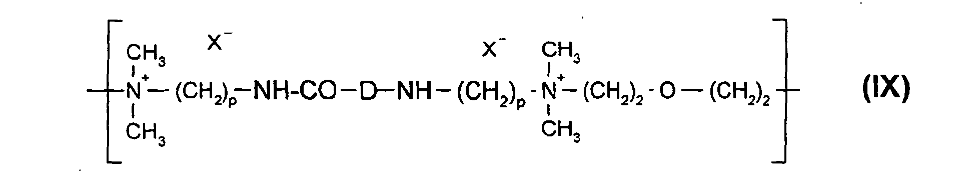 dans laquelle p désigne un nombre entier variant de 1 à 6 environ, D peut être nul ou peut représenter un groupement -(CH₂)ᵣ -CO- dans lequel r désigne un nombre égal à 4 ou à 7, X⁻ est un anion ;
   De tels polymères peuvent être préparés selon les procédés décrits dans les brevets U.S.A. n° 4 157 388, 4 702 906, 4 719 282. Ils sont notamment décrits dans la demande de brevet EP-A-122 324.
   Parmi eux, on peut par exemple citer, les produits "Mirapol A 15", "Mirapol AD1", "Mirapol AZ1" et "Mirapol 175" vendus par la société Miranol.
(**12**) Les polymères quaternaires de vinylpyrrolidone et de vinylimidazole tels que par exemple les produits commercialisés sous les dénominations Luviquat FC 905, FC 550 et FC 370 par la société B.A.S.F.
(**13**) Les polyamines comme le Polyquart H vendu par HENKEL, référencé sous le nom de "POLYETHYLENEGLYCOL (15) TALLOW POLYAMINE " dans le dictionnaire CTFA.
(**14**) Les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE^{®} SC 92 " par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE^{®} SC 95 " et " SALCARE^{®} SC 96 " par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les polymères des familles (1), (9), (10) (11) et (14) et encore plus préférentiellement les polymères aux motifs récurrents de formules (W) et (U) suivantes : 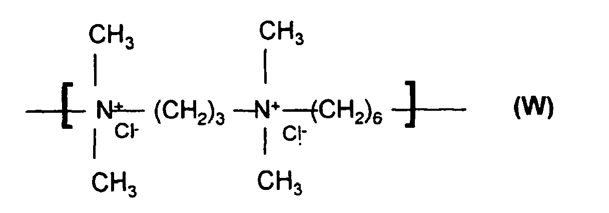 et notamment ceux dont le poids moléculaire, déterminé par chromatographie par perméation de gel, est compris entre 9500 et 9900; 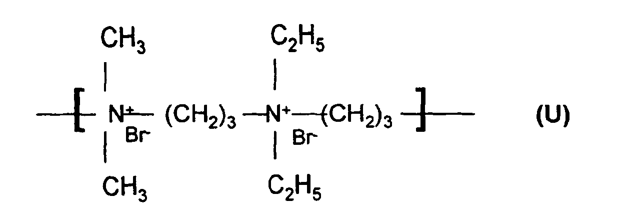 et notamment ceux dont le poids moléculaire, déterminé par chromatographie par perméation de gel, est d'environ 1200.

La concentration en polymère cationique dans la composition selon la présente invention peut varier de 0,01 à 10% en poids par rapport au poids total de la composition, de préférence de 0,05 à 5% et plus préférentiellement encore de 0,1 à 3%.

### Polymères amphotères

Les polymères amphotères utilisables conformément à la présente invention peuvent être choisis parmi les polymères comportant des motifs K et M répartis statistiquement dans la chaîne polymère, où K désigne un motif dérivant d'un monomère comportant au moins un atome d'azote basique et M désigne un motif dérivant d'un monomère acide comportant un ou plusieurs groupements carboxyliques ou sulfoniques, ou bien K et M peuvent désigner des groupements dérivant de monomères zwittérioniques de carboxybétaïnes ou de sulfobétaïnes; K et M peuvent également désigner une chaîne polymère cationique comportant des groupements amine primaire, secondaire, tertiaire ou quaternaire, dans laquelle au moins l'un des groupements amine porte un groupement carboxylique ou sulfonique relié par l'intermédiaire d'un radical hydrocarboné, ou bien K et M font partie d'une chaîne d'un polymère à motif éthylène α,β-dicarboxylique dont l'un des groupements carboxyliques a été amené à réagir avec une polyamine comportant un ou plusieurs groupements amine primaire ou secondaire.

Les polymères amphotères répondant à la définition donnée ci-dessus plus particulièrement préférés sont choisis parmi les polymères suivants :
(1) Les polymères résultant de la copolymérisation d'un monomère dérivé d'un composé vinylique portant un groupement carboxylique tel que plus particulièrement l'acide acrylique, l'acide méthacrylique, l'acide maléique, l'acide alpha-chloracrylique, et d'un monomère basique dérivé d'un composé vinylique substitué contenant au moins un atome basique tel que plus particulièrement les dialkylaminoalkylméthacrylate et acrylate, les dialkylaminoalkylméthacrylamide et acrylamide. De tels composés sont décrits dans le brevet américain n° 3 836 537. On peut également citer le copolymère acrylate de sodium / chlorure d'acrylamidopropyl trimethyl ammonium vendu sous la dénomination POLYQUART KE 3033 par la Société HENKEL.
   Le composé vinylique peut être également un sel de dialkyldiallylammonium tel que le chlorure de diméthyldiallylammonium. Les copolymères d'acide acrylique et de ce dernier monomère sont proposés sous les appellations MERQUAT 280, MERQUAT 295 et MERQUAT PLUS 3330 par la société CALGON.
(2) Les polymères comportant des motifs dérivant :
   a) d'au moins un monomère choisi parmi les acrylamides ou les méthacrylamides substitués sur l'azote par un radical alkyle,
   b) d'au moins un comonomère acide contenant un ou plusieurs groupements carboxyliques réactifs, et
   c) d'au moins un comonomère basique tel que des esters à substituants amine primaire, secondaire, tertiaire et quaternaire des acides acrylique et méthacrylique et le produit de quaternisation du méthacrylate de diméthylamino-éthyle avec le sulfate de diméthyle ou diéthyle.
      Les acrylamides ou méthacrylamides N-substitués plus particulièrement préférés selon l'invention sont les groupements dont les radicaux alkyle contiennent de 2 à 12 atomes de carbone et plus particulièrement le N-éthylacrylamide, le N-tertiobutyl-acrylamide, le N-tertiooctyl-acrylamide, le N-octylacrylamide, le N-décylacrylamide, le N-dodécylacrylamide ainsi que les méthacrylamides correspondants.
      Les comonomères acides sont choisis plus particulièrement parmi les acides acrylique, méthacrylique, crotonique, itaconique, maléique, fumarique ainsi que les monoesters d'alkyle ayant 1 à 4 atomes de carbone des acides ou des anhydrides maléique ou fumarique.
      Les comonomères basiques préférés sont des méthacrylates d'aminoéthyle, de butyl aminoéthyle, de N,N'-diméthylaminoéthyle, de N-tertio-butylaminoéthyle. On utilise particulièrement les copolymères dont la dénomination CTFA (4ème Ed., 1991) est Octylacrylamide/acrylates/butylaminoethylmethacrylate copolymer tels que les produits vendus sous la dénomination AMPHOMER ou LOVOCRYL 47 par la société NATIONAL STARCH.
(3) Les polyaminoamides réticulés et alcoylés partiellement ou totalement dérivant de polyaminoamides de formule générale :

   ⁅CO-R₁₉-CO-Z⁆ (X)

   dans laquelle R₁₉ représente un radical divalent dérivé d'un acide dicarboxylique saturé, d'un acide aliphatique mono ou dicarboxylique à double liaison éthylénique, d'un ester d'un alcanol inférieur ayant 1 à 6 atomes de carbone de ces acides ou d'un radical dérivant de l'addition de l'un quelconque desdits acides avec une amine bis primaire ou bis secondaire, et Z désigne un radical d'une polyalkylène-polyamine bis-primaire, mono ou bis-secondaire et de préférence représente :
   a) dans les proportions de 60 à 100 moles %, le radical 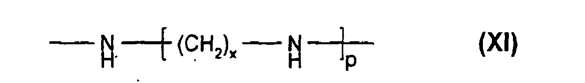 où x=2 et p=2 ou 3, ou bien x=3 et p=2
      ce radical dérivant de la diéthylène triamine, de la triéthylène tétraamine ou de la dipropylène triamine;
   b) dans les proportions de 0 à 40 moles % le radical (XI) ci-dessus, dans lequel x=2 et p=1 et qui dérive de l'éthylènediamine, ou le radical dérivant de la pipérazine : 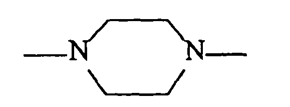
   c) dans les proportions de 0 à 20 moles % le radical -NH-(CH₂)₆-NH-dérivant de l'hexaméthylènediamine, ces polyaminoamines étant réticulées par addition d'un agent réticulant bifonctionnel choisi parmi les épihalohydrines, les diépoxydes, les dianhydrides, les dérivés bis insaturés, au moyen de 0,025 à 0,35 mole d'agent réticulant par groupement amine du polyaminoamide et alcoylés par action d'acide acrylique, d'acide chloracétique ou d'une alcane sultone ou de leurs sels.
      Les acides carboxyliques saturés sont choisis de préférence parmi les acides ayant 6 à 10 atomes de carbone tels que l'acide adipique, triméthyl-2,2,4-adipique et triméthyl-2,4,4-adipique, téréphtalique, les acides à double liaison éthylénique comme par exemple les-acides acrylique, méthacrylique, itaconique.
      Les alcanes sultanes utilisées dans l'alcoylation sont de préférence la propane ou la butane sultone, les sels des agents d'alcoylation sont de préférence les sels de sodium ou de potassium.
(4) Les polymères comportant des motifs zwittérioniques de formule : 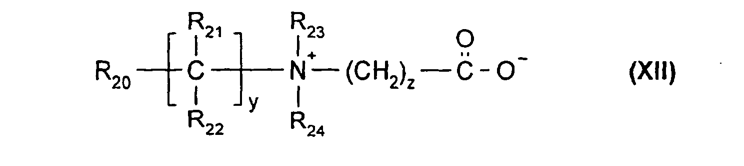 dans laquelle R₂₀ désigne un groupement insaturé polymérisable tel qu'un groupement acrylate, méthacrylate, acrylamide ou méthacrylamide, y et z représentent un nombre entier de 1 à 3, R₂₁ et R₂₂ représentent un atome d'hydrogène, méthyle, éthyle ou propyle, R₂₃ et R₂₄ représentent un atome d'hydrogène ou un radical alkyle de telle façon que la somme des atomes de carbone dans R₂₃ et R₂₄ ne dépasse pas 10.
   Les polymères comprenant de telles unités peuvent également comporter des motifs dérivés de monomères non zwittérioniques tels que l'acrylate ou le méthacrylate de diméthyl ou diéthylaminoéthyle ou des alkyle acrylates ou méthacrylates, des acrylamides ou méthacrylamides ou l'acétate de vinyle.
   A titre d'exemple, on peut citer le copolymère de méthacrylate de méthyle / diméthyl-carboxyméthylammonio-éthylméthacrylate de méthyle tel que le produit vendu sous la dénomination DIAFORMER Z301 par la société SANDOZ.
(5) les polymères dérivés du chitosane comportant des motifs monomères répondant aux formules (XIII), (XIV), (XV) suivantes : 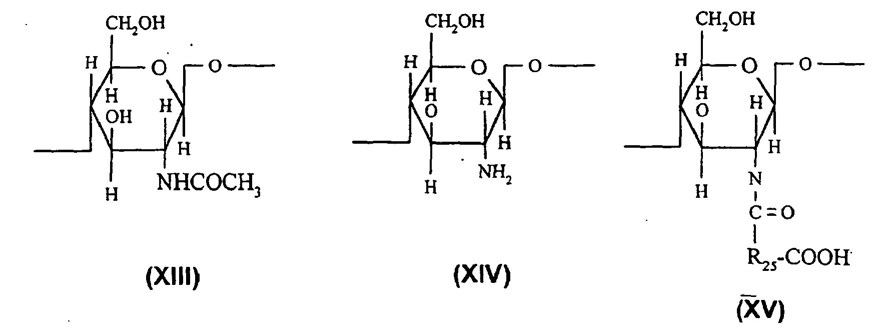 le motif (XIII) étant présent dans des proportions comprises entre 0 et 30%, le motif (XIV) dans des proportions comprises entre 5 et 50% et le motif F dans des proportions comprises entre 30 et 90%, étant entendu que dans ce motif (XV), R₂₅ représente un radical de formule : 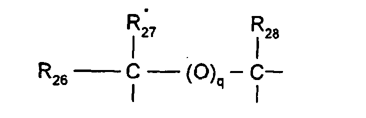 dans laquelle
   si q=0, R₂₆, R₂₇ et R₂₈, identiques ou différents, représentent chacun un atome d'hydrogène, un reste méthyle, hydroxyle, acétoxy ou amino, un reste monoalcoylamine ou un reste dialcoylamine éventuellement interrompus par un ou plusieurs atomes d'azote et/ou éventuellement substitués par un ou plusieurs groupes amine, hydroxyle, carboxyle, alcoylthio, sulfonique, un reste alcoylthio dont le groupe alcoyle porte un reste amino, l'un au moins des radicaux R₂₆, R₂₇ et R₂₈ étant dans ce cas un atome d'hydrogène ;
   ou si q=1, R₂₆, R₂₇ et R₂₈ représentent chacun un atome d'hydrogène, ainsi que les sels formés par ces composés avec des bases ou des acides.
(6) Les polymères dérivés de la N-carboxyalkylation du chitosane comme le N-carboxyméthyl chitosane ou le N-carboxybutyl chitosane vendu sous la dénomination "EVALSAN" par la société JAN DEKKER.
(7) Les polymères répondant à la formule générale (XI) tels que ceux décrits par exemple dans le brevet français 1 400 366 : 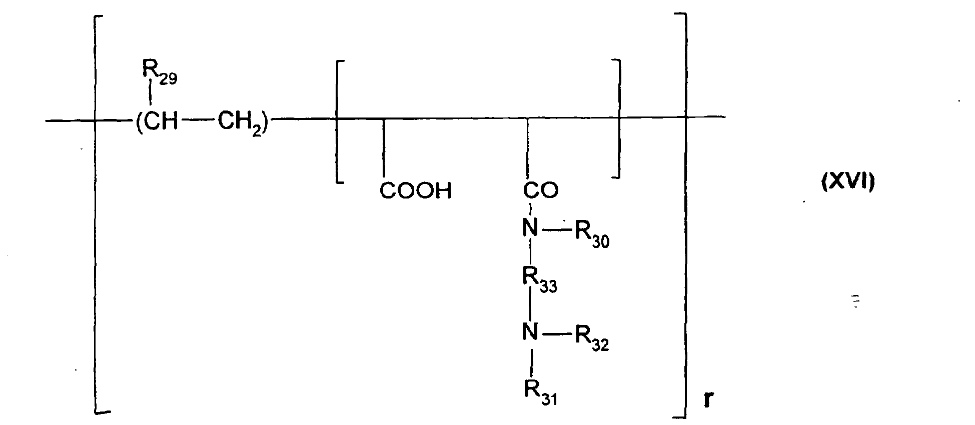 dans laquelle R₂₉ représente un atome d'hydrogène, un radical CH₃O, CH₃CH₂O, phényle, R₃₀ désigne l'hydrogène ou un radical alkyle inférieur tel que méthyle, éthyle, R₃₁désigne l'hydrogène ou un radical alkyle inférieur tel que méthyle, éthyle, R₃₂ désigne un radical alkyle inférieur tel que méthyle, éthyle ou un radical répondant à la formule : -R₃₃-N(R₃₁)₂, R₃₃ représentant un groupement -CH₂-CH₂- , -CH₂-CH₂-CH₂- , -CH₂-CH(CH₃)- , R₃₁ ayant les significations mentionnées ci-dessus,
   ainsi que les homologues supérieurs de ces radicaux et contenant jusqu'à 6 atomes de carbone.
(8) Des polymères amphotères du type -D-X-D-X- choisis parmi:
   a) les polymères obtenus par action de l'acide chloracétique ou le chloracétate de sodium sur les composés comportant au moins un motif de formule :

      -D-X-D-X-D- (XVII)

      où D désigne un radical 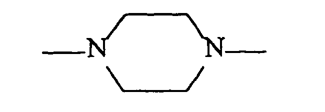 et X désigne le symbole E ou E', E ou E' identiques ou différents désignent un radical bivalent qui est un radical alkylène à chaîne droite ou ramifiée comportant jusqu'à 7 atomes de carbone dans la chaîne principale non substituée ou substituée par des groupements hydroxyle et pouvant comporter en outre des atomes d'oxygène, d'azote, de soufre, 1 à 3 cycles aromatiques et/ou hétérocycliques; les atomes d'oxygène, d'azote et de soufre étant présents sous forme de groupements éther, thioéther, sulfoxyde, sulfone, sulfonium, alkylamine, alkénylamine, des groupements hydroxyle, benzylamine, oxyde d'amine, ammonium quaternaire, amide, imide, alcool, ester et/ou uréthanne ;
   b) les polymères de formule :

      -D-X-D-X- (XVIII)
   où D désigne un radical 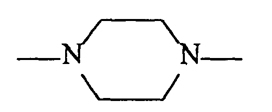 et X désigne le symbole E ou E' et au moins une fois E'; E ayant la signification indiquée ci-dessus et E' est un radical bivalent qui est un radical alkylène à chaîne droite ou ramifiée ayant jusqu'à 7 atomes de carbone dans la chaîne principale, substitué ou non par un ou plusieurs radicaux hydroxyle et comportant un ou plusieurs atomes d'azote, l'atome d'azote étant substitué par une chaîne alkyle interrompue éventuellement par un atome d'oxygène et comportant obligatoirement une ou plusieurs fonctions carboxyle ou une ou plusieurs fonctions hydroxyle et bétaïnisées par réaction avec l'acide chloracétique ou du chloracétate de soude.
(9) Les copolymères alkyl(C₁-C₅)vinyléther / anhydride maléique modifié partiellement par semiamidification avec une N,N-dialkylaminoalkylamine telle que la N,N-diméthylaminopropylamine ou par semiestérification avec une N,N-dialcanolamine. Ces copolymères peuvent également comporter d'autres comonomères vinyliques tels que le vinylcaprolactame.

Les polymères amphotères particulièrement préférés selon l'invention sont ceux de la famille (1).

Selon l'invention, le ou les polymères amphotères peuvent représenter de 0,01 % à 10 % en poids, de préférence de 0,05 % à 5 % en poids, et encore plus préférentiellement de 0,1 % à 3 % en poids, du poids total de la composition.

La composition prête à l'emploi selon l'invention comprend de préférence dans la composition colorante (A) et/ou dans la composition oxydante (B) un ou plusieurs tensioactifs additionnels.
Le ou les tensioactifs additionnels peuvent être indifféremment choisis, seuls ou en mélanges, au sein des tensioactifs anioniques, amphotères, non ioniques, zwittérioniques et cationiques.

Les tensioactifs additionnels convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, ou les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensio-actifs non-ioniques rentrant particulièrement bien dans le cadre de la présente invention tout comme les alcools gras éthoxylés.

### (iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₃₄ -CONHCH₂CH₂ -N(R₃₅)(R₃₆)(CH₂COO⁻)

dans laquelle : R₃₄ désigne un radical alkyle d'un acide R₃₄-COOH présent dans l'huile de coprah hydrolysée, un radical heptyle, nonyle ou undécyle, R₃₅ désigne un groupement bêta-hydroxyéthyle et R₃₆ un groupement carboxyméthyle ;
et

R₃₄'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₃₄' désigne un radical alkyle d'un acide R₃₇ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C17 insaturé.

Ces composés sont classés dans le dictionnaire CTFA, 5ème édition, 1993, sous les dénominations Disodium Cocoamphodiacetate, Disodium Lauroampho-diacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroampho-dipropionic acid, Cocoamphodipropionic acid.
A titre d'exemple on peut citer le cocoamphodiacetate commercialisé sous la dénomination commerciale MIRANOL^{®} C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Les quantités d'agents tensioactifs additionnels présents dans la composition selon l'invention peuvent varier de 0,01 à 40% et de préférence de 0,1 à 30% du poids total de la composition.

La composition prête à l'emploi selon l'invention peut également contenir dans la composition colorante (A) et/ou la composition oxydante (B) d'autres agents d'ajustement de la rhéologie tels que les épaississants cellulosiques (hydroxyéthycellulose, hydroxypropylcellulose, carboxyméthylcellulose..), la gomme de guar et ses dérivés( hydroxypropylguar..), les gommes d'origine microbienne (gomme de xanthane, gomme de scléroglucane..), les épaississants synthétiques ne possédant pas une chaîne grasse tels que les homopolymères réticulés d'acide acrylique ou d'acide acrylamidopropanesulfonique.
Ces épaississants d'appoint peuvent représenter de 0,01 à 10% en poids du poids total de la composition.

Le milieu de la composition approprié pour la teinture est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

La composition selon l'invention peut encore contenir une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, organomodifiées (notamment par des groupements amines) ou non, des conservateurs, des céramides, des pseudocéramides, des huiles végétales, minérales ou de synthèse, les vitamines ou provitamines comme le panthénol, des opacifiants, etc....

Ladite composition peut également contenir des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butyl-hydroquinone et l'acide homogentisique, et ils sont alors généralement présents dans des quantités allant d'environ 0,05 à 3% en poids par rapport au poids total de la composition.

La composition selon l'invention peut également contenir un ou plusieurs alcools gras, ces alcools gras étant introduits sous forme pure ou de mélange. On peut citer parmi eux plus particulièrement les alcools laurique, cétylique, stéarylique, oléique et leurs mélanges. Ces alcools gras additonnels peuvent représenter de 0,001 à 20% en poids environ du poids total de la composition.

Bien entendu, l'homme de l'art veillera à choisir le ou les éventuels composés complémentaires mentionnés ci-avant, de manière telle que les propriétés avantageuses attachées intrinsèquement à la composition prête à l'emploi selon l'invention ne soient pas, ou substantiellement pas, altérées par la ou les adjonctions envisagées.

Dans la composition oxydante (B), l'agent oxydant est choisi de préférence parmi le peroxyde d'hydrogène, le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.
On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition colorante (A) ou de la composition prête à l'emploi et appliquée sur les fibres kératiniques [composition résultant du mélange de la composition tinctoriale (A) et de la composition oxydante (B)], est généralement compris entre les valeurs 4 et 12. Il est de préférence compris entre 6 et 11, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium
ou de potassium et les composés de formule (XIX) suivante : 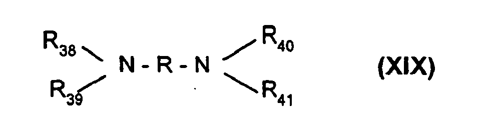 dans laquelle R est un reste propylène éventuellement substitué par un groupement hydroxyle ou un radical alkyle en C₁-C₄ ; R₃₈, R₃₉, R₄₀ et R₄₁, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

Le procédé de teinture selon l'invention consiste, de préférence, à appliquer la composition prête à l'emploi, réalisée extemporanément au moment de l'emploi à partir des compositions (A) et (B) décrites ci-avant, sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Une variante de ce procédé consiste à prendre une composition A' contenant au moins un colorant d'oxydation et au moins un alcool gras mono- ou poly-glycérolé mais sans polymère épaississant à chaîne grasse, une composition A" contenant au moins un polymère épaississant à chaîne grasse, et à mélanger au moment de l'emploi les compositions A' et A" avec la composition B, puis à appliquer et laisser agir le mélange comme dans le cas précédent.

Des exemples concrets illustrant l'invention sont indiqués ci-après.

### EXEMPLES

On a préparé les compositions suivantes :

### Composition oxydante :

| | |
|---|---|
| Alcool gras | 2,3 g |
| Alcool gras oxyéthyléné | 0,6 g |
| Amide grasse | 0,9 g |
| Glycérine | 0,5 g |
| Peroxyde d'hydrogène | 7,5 g |
| parfum | qs |
| Eau déminéralisée qsp | 100 g |

### Composition colorante :

(exprimée en grammes)

| | |
|---|---|
| Mélange d'alcools linéaires en C18 à C24 [C18/C20/C22/C24, 7/58/30/6, teneur en alcools>95%] (NAFOL 20-22) | 3 |
| Mélange d'alcools linéaires en C18 à C24 [C18/C20/C22/C24, 7/58/3016, teneur en alcools>95%] polyglycérolés à 6 moles de glycérol | 1,35 |
| Alcool cétéarylique polyglycérolé à 2 moles de glycérol | 4 |
| Alcool cétéarylique polyglycérolé à 6 moles de glycérol | 2 |
| Acide oléique | 2,6 |
| Distéarate de glycol | 2 |
| Propylène glycol | 5 |
| Monoisopropanolamide d'acides de coprah | 2 |
| Aculyn 44 vendu par la société ROHM & HAAS | 1,4 MA* |
| Acide polyacrylique réticulé | 0,6 |
| Polymère cationique de formule (W) | 3 MA* |
| Merquat 100 vendu par la société CALGON | 0,4 MA* |
| Réducteurs | 0,7 |
| Séquestrants | 0,2 |
| 1,3-dihydroxybenzène (résorcinol) | 0,6 |
| 1,4-diaminobenzène | 0,5 |
| 1-hydroxy-3-amino-benzène | 0,1 |
| 1-hydroxy-2-amino-benzène | 0,05 |
| 1-hydroxy-4-amino-benzène | 0,09 |
| 6-hydroxy-benzomorpholine | 0,017 |
| 1-β-hydroxyéthyloxy-2,4-diamino-benzène, dichlorhydrate | 0,039 |
| Monobutyléther de propylène glycol | 2,5 |
| Monoéthanolamine pure | 1,06 |
| Ammoniaque (à 20,5% en ammoniac) | 11,1 |
| Eau qsp | 100 |

| | |
|---|---|
| MA*= Matière Active | |

La composition colorante a été mélangée, au moment de l'emploi, dans un bol en plastique et pendant 2 minutes, à la composition oxydante donnée ci-dessus, à raison de 1 partie de composition colorante pour 1,5 partie de composition oxydante.
On a appliqué le mélange obtenu sur des mèches de cheveux naturels à 90% de blancs et on a laissé poser 30 minutes.
On a ensuite rincé les mèches à l'eau, on les a lavées au shampooing, à nouveau rincées à l'eau, puis séchées et démélées.
Les cheveux ont alors été teints dans une nuance chatain clair puissante.

Des résultats du même type ont été obtenus en remplaçant dans l'exemple ci-dessus, l'Aculyn 44 par 0,5%MA de Quatrisoft LM 200 vendu par la société AMERCHOL ou par 0,5%MA de Pure Thix HH vendu par la société SUD CHEMIE.

## Revendications

1. Composition dîte composition (A) pour la teinture d'oxydation des fibres kératiniques, en particulier des fibres kératiniques humaines telles que les cheveux, comprenant, dans un milieu approprié pour la teinture, au moins un colorant d'oxydation,
**caractérisée par le fait qu'**elle contient en outre au moins un polymère épaississant comportant au moins une chaîne grasse et au moins un alcool gras mono- ou poly- glycérolé.

2. Composition selon la revendication 1, **caractérisée par le fait que** l'alcool gras mono- ou poly- glycérolé est de structure suivante :
RO- (-CH₂-CH(CH₂OH)-O-)ₙ-H
dans laquelle :
R représente un radical saturé ou insaturé, linéaire ou ramifié, comportant de 8 à 40 atomes de carbone ;
n représente un nombre compris entre 1 et 30.

3. Composition selon la revendication 2, **caractérisée par le fait que** dans la formule, R comporte de 10 à 30 atomes de carbone.

4. Composition selon la revendication 2, **caractérisée par le fait que** dans la formule, n représente un nombre compris entre 1 et 10.

5. Composition selon l'une quelconque des revendications 1 à 4, **caractérisée par le fait que** le ou les alcools gras mono- ou poly- glycérolés représentent en poids de 0,01 à 30%, de préférence de 0,05 à 20% et plus préférentiellement de 0,1 à 15% du poids total de la composition.

6. Composition selon l'une quelconque des revendications 1 à 5, **caractérisée par le fait que** le polymère épaississant comportant au moins une chaîne grasse est anionique, non ionique ou cationique.

7. Composition selon la revendication 6, **caractérisée par le fait que** le polymère épaississant anionique à chaîne grasse comporte au moins un motif hydrophile et au moins un motif éther d'allyle à chaîne grasse.

8. Composition selon la revendication 7, **caractérisée par le fait que** le motif hydrophile est un monomère anionique insaturé éthylénique et de préférence un acide carboxylique vinylique.

9. Composition selon la revendication 7, **caractérisée par le fait que** le motif éther d'allyle à chaîne grasse est un monomère de formule (I) suivante :
CH₂ = C R' CH₂ O Bₙ R (I)
dans laquelle R' est H ou CH₃, B est éthylèneoxy, n est nul ou désigne un entier allant de 1 à 100, R est un radical hydrocarboné choisi parmi les radicaux alkyl, arylalkyle, aryle, alkylaryle, cycloalkyle, comprenant 8 à 30 atomes de carbone, de préférence 10 à 24, et plus particulièrement 12 à 18 atomes de carbone.

10. Composition selon la revendication 6, **caractérisée par le fait que** le polymère épaississant anionique à chaîne grasse comporte au moins un motif hydrophile de type acide carboxylique insaturé oléfinique et au moins un motif hydrophobe de type ester d'alkyl (C₁₀-C₃₀) d'acide carboxylique insaturé.

11. Composition selon la revendication 10, **caractérisée par** le fait le motif hydrophile de type acide carboxylique insaturé oléfinique est un monomère de formule (II) suivante : 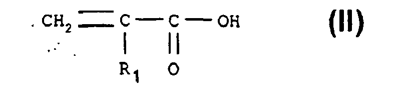 dans laquelle, R₁ désigne H ou CH₃ ou C₂H₅, et dont le motif hydrophobe de type ester d'alkyl (C₁₀-C₃₀) d'acide carboxylique insaturé est un monomère de formule (III) suivante : 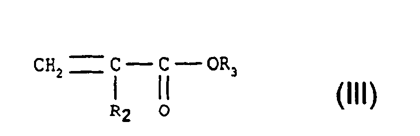 dans laquelle, R₂ désigne H ou CH₃ ou C₂H₅, R₃ désignant un radical alkyle en C₁₀-C₃₀, et de préférence en C₁₂-C₂₂.

12. Composition selon la revendication 6, **caractérisée par le fait que** le polymère épaississant anionique à chaîne grasse est un terpolymère d'anhydride maléique/α-oléfine en C₃₀-C₃₈/ maléate d'alkyle.

13. Composition selon la revendication 6, **caractérisée par le fait que** le polymère épaississant anionique à chaîne grasse est un terpolymère acrylique comprenant :
(a) un acide carboxylique à insaturation α,β-monoéthylénique
(b) un monomère à insaturation α,β-monoéthylénique non-tensio-actif différent de (a)
(c) un mono-uréthane non-ionique produit de la réaction d'un tensio-actif monohydrique avec un monoisocyanate à insaturation monoéthylénique.

14. Composition selon la revendication 6, **caractérisée par le fait que** le polymère épaississant anionique à chaîne grasse est un copolymère d'un acide carboxylique à insaturation α,β-monoéthylénique et d'un ester d'acide carboxylique à insaturation α,β-monoéthylénique et d'alcool gras oxyalkyléné.

15. Composition selon la revendication 6, **caractérisée par le fait que** le polymère épaississant non-ionique à chaîne grasse est choisi dans le groupe formé par :
(1) les celluloses modifiées par des groupements comportant au moins une chaîne grasse ;
(2) les hydroxypropylguars modifiés par des groupements comportant au moins une chaîne grasse;
(3) les polyuréthanes polyéthers comportant dans leur chaîne, à la fois des séquences hydrophiles de nature polyoxyéthylénée et des séquences hydrophobes qui sont des enchaînements aliphatiques seuls et/ou des enchaînements cycloaliphatiques et/ou aromatiques ;
(4) les copolymères de vinyl pyrrolidone et de monomères hydrophobes à chaîne grasse ;
(5) les copolymères de méthacrylates ou d'acrylates d'alkyles en C₁-C₆ et de monomères amphiphiles comportant au moins une chaîne grasse;
(6) les copolymères de méthacrylates ou d'acrylates hydrophiles et de monomères hydrophobes comportant au moins une chaîne grasse;
(7) les polymères à squelette aminoplaste éther comportant au moins une chaîne grasse.

16. Composition selon la revendication 15, **caractérisée par le fait que** le polyéther polyuréthane comporte au. moins deux chaînes lipophiles hydrocarbonées, ayant de C₆ à C₃₀ atomes de carbone, séparées par une séquence hydrophile, les chaînes hydrocarbonées étant des chaînes pendantes ou des chaînes en bout de séquence hydrophile.

17. Composition selon la revendication 15, **caractérisée par le fait que** le polyéther polyuréthane peut être multiséquencé en particulier sous forme de triblocs.

18. Composition selon la revendication 6, **caractérisée par le fait que** le polymère épaississant cationique à chaîne grasse est un dérivé de cellulose quaternisée ou un polyacrylate à groupements latéraux aminés non cycliques.

19. Composition selon l'une quelconque des revendications 1 à 18 **caractérisée par le fait que** le ou les polymères épaississants à chaîne grasse sont présents à raison de 0,01 à 10% et de préférence de 0,1 à 5% en poids du poids total de la composition.

20. Composition selon l'une quelconque des revendications précédentes, **caractérisée par le fait que** le colorant d'oxydation est choisi parmi les bases d'oxydation et les coupleurs.

21. Composition selon l'une quelconque des revendications précédentes, **caractérisée par le fait qu'**elle contient au moins une base d'oxydation.

22. Composition selon les revendications 20 ou 21, **caractérisée par le fait que** les bases d'oxydation sont choisis parmi les ortho- ou para- phénylènediamines, les bases doubles, les ortho- ou para- aminophénols, et les bases hétérocycliques, ainsi que les sels d'addition de ces composés avec un acide.

23. Composition selon la revendication 22, **caractérisée par le fait que** les para-phénylènediamines sont choisies parmi les composés de structure (I) suivante : 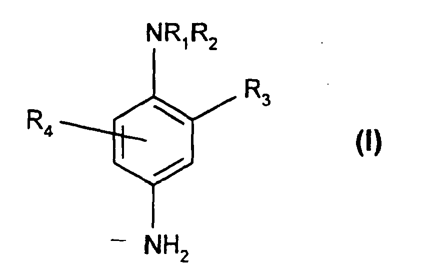 dans laquelle :
R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), alkyle en C₁-C₄ substitué par un groupement azoté, phényle ou 4'-aminophényle ;
R₂ représente un atome d'hydrogène, un radical alkyle en C₁-₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ;
R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄,
R₄ représente un atome d'hydrogène, d'halogène ou un radical alkyle en C₁-C₄.

24. Composition selon la revendication 22, **caractérisée par le fait que** les bases doubles sont choisies parmi les composés de structure (II) suivante : 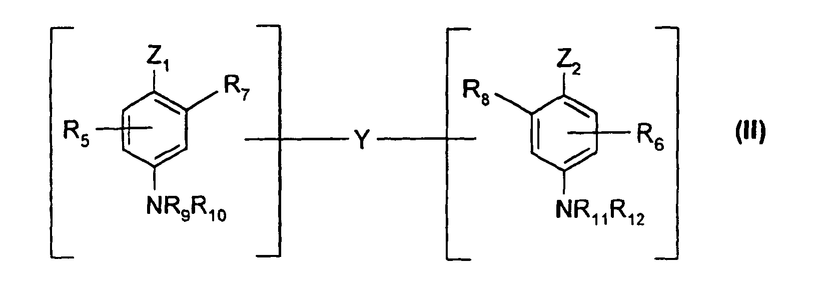 dans laquelle :
- Z₁ et Z₂, identiques ou différents, représentent un radical hydroxyle ou -NH₂ pouvant être substitué par un radical alkyle en C₁-C₄ ou par un bras de liaison Y ;
- le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
- R₇, R₈, R₉, R₁₀, R₁₁ et R₁₂, identiques ou différents, représentent un atome d'hydrogène, un bras de liaison Y ou un radical alkyle en C₁-C₄ ;
étant entendu que les composés de formule (II) ne comportent qu'un seul bras de liaison Y par molécule.

25. Composition selon les revendications 23 ou 24, **caractérisée par le fait que** les groupements azotés sont choisis parmi les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

26. Composition selon la revendication 22, **caractérisée par le fait que** les para-aminophénols sont choisis parmi les composés de structure (III) suivante: 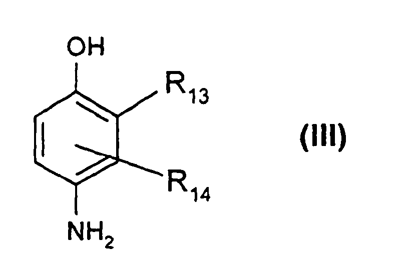 dans laquelle :
R₁₃ représente un atome d'hydrogène,un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

27. Composition selon la revendication 22, **caractérisée par le fait que** les bases hétérocycliques sont choisies parmi les dérivés pyridiniques, les dérivés pyrimidiniques dont les pyrazolopyrimidines, et les dérivés pyrazoliques.

28. Composition selon les revendications 20 à 27, **caractérisée par le fait que** les bases d'oxydation sont présentes dans des concentrations allant de 0,0005 à 12% en poids par rapport au poids total de la composition.

29. Composition selon les revendications 20 ou 21 , **caractérisée par le fait que** les coupleurs sont choisis parmi les métaphénylènediamines, les métaaminophénols, les métadiphénols, les coupleurs hétérocycliques, et les sels d'addition de ces composés avec un acide.

30. Composition selon les revendications 20, 21 ou 29, **caractérisée par le fait que** les coupleurs sont présents dans des concentrations allant de 0,0001 à 10% en poids par rapport au poids total de la composition.

31. Composition selon les revendications 22 ou 29, **caractérisée par le fait que** les sels d'addition avec un acide bases d'oxydation et des coupleurs sont choisis parmi les chlorhydrates, les bromhydrates, les sulfates, les tartrates, les lactates et les acétates.

32. Composition selon l'une quelconque des revendications précédentes, **caractérisée par le fait qu'**elle contient en outre des colorants directs.

33. Composition selon l'une quelconque des revendications précédentes, **caractérisée par le fait qu'**elle contient en outre au moins un agent réducteur, dans des quantités allant de 0,05 à 3% en poids par rapport au poids total de la composition.

34. Composition selon l'une quelconque des revendications précédentes, **caractérisée par le fait qu'**elle contient en outre un ou plusieurs alcools gras.

35. Composition selon la revendication 34, **caractérisée par le fait que** le ou les alcools gras représentent en poids de 0,001 à 20% du poids total de la composition.

36. Composition prête à l'emploi pour la teinture d'oxydation des fibres kératiniques en particulier des fibres kératiniques humaines telles que les cheveux **caractérisée par le fait qu'**elle est obtenue par mélange d'une composition (A) telle que définie à l'une quelconque des revendications 1 à 35 et d'une composition (B) contenant au moins un agent oxydant.

37. Composition selon la revendication 36, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction telles que les laccases, les peroxydases et les oxydoréductases à 2 électrons, le cas échéant en présence de leur donneur ou cofacteur respectif.

38. Composition selon la revendication 37, **caractérisée par le fait que** l'agent oxydant est le peroxyde d'hydrogène.

39. Composition selon la revendication 38, **caractérisée par le fait que** l'agent oxydant est une solution d'eau oxygénée dont le titre varie de 1 à 40 volumes.

40. Composition selon l'une quelconque des revendications 1 à 39, **caractérisée par le fait qu'**elle possède un pH allant de 4 à 12.

41. Composition selon la revendication 36, **caractérisée par le fait que** la composition (A) et/ou la composition (B) contient au moins un polymère cationique ou amphotère.

42. Composition selon la revendication 41, **caractérisée par le fait que** le polymère cationique est un polyammonium quaternaire constitué de motifs récurrents répondant à la formule (W) suivante :

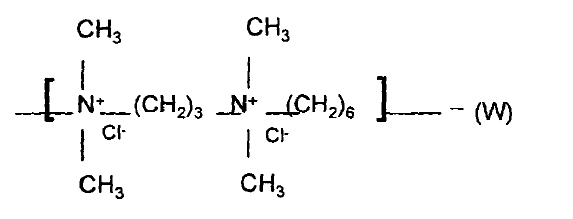

43. Composition selon la revendication 41, **caractérisée par le fait que** le polymère cationique est un polyammonium quaternaire constitué de motifs récurrents répondant à la formule (U) suivante :

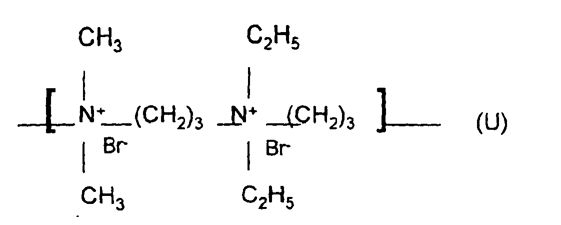

44. Composition selon la revendication 41, **caractérisé par le fait que** le polymère amphotère est un copolymère comprenant au moins comme monomère de l'acide acrylique et un sel de diméthyldiallylammonium.

45. Composition selon l'une quelconque des revendications 41 à 44, **caractérisée par le fait que** le ou les polymères cationiques ou amphotères représentent de 0,01% à 10%, de préférence de 0,05% à 5%, et plus préférentiellement de 0,1 % à 3% en poids, du poids total de la composition.

46. Composition selon la revendication 36, **caractérisée par le fait que** la composition (A) et/ou la composition (B) contient au moins un tensioactif additionnel choisi parmi les tensioactifs anioniques, cationiques, non ioniques ou amphotères.

47. Composition selon la revendication 46, **caractérisée par le fait que** les agents tensioactifs additionnels représentent 0,01 à 40% et de préférence 0,1 à 30% du poids total de la composition.

48. Composition selon la revendication 36, **caractérisée par le fait que** la composition (A) et/ou la composition (B) contient un agent épaississant choisi parmi les dérivés de cellulose, les dérivés de guar, les gommes d'origine microbienne, les épaississants synthétiques ne possédant pas une chaîne grasse.

49. Composition selon la revendication 48, **caractérisée par le fait que** le ou les agents épaississants représentent de 0,01 à 10% en poids du poids total de la composition.

50. Procédé de teinture des fibres kératiniques et en particulier des fibres kératiniques humaines telles que les cheveux, **caractérisé par le fait qu'**il consiste à appliquer sur les fibres une composition de teinture (A) telle que définie à l'une quelconque des revendications 1 à 35, et 40 à 49 et à révéler la couleur en milieu alcalin, neutre ou acide à l'aide d'une composition (B) contenant au moins un agent oxydant, ladite composition (B) étant définie à l'une quelconque des revendications 36 à 49 et ajoutée juste au moment de l'emploi à la composition (A) ou appliquée séquentiellement sans rinçage intermédiaire.

51. Procédé de teinture des fibres kératiniques et en particulier des fibres kératiniques humaines telles que les cheveux, **caractérisé par le fait qu'**il consiste à appliquer sur les fibres une composition colorante contenant dans un milieu approprié pour la teinture au moins un colorant d'oxydation, au moins un alcool gras mono- ou poly- glycérolé, en la présence ou en l'absence d'un polymère épaississant comportant au moins une chaîne grasse tel que défini à l'une quelconque des revendications 6 à 19, et à révéler la couleur en milieu alcalin, neutre ou acide à l'aide d'une composition oxydante contenant un agent oxydant et une quantité efficace d'au moins un polymère épaississant comportant au moins une chaîne grasse tel que défini à l'une quelconque des revendications 6 à 19, et qui est mélangée juste au moment de l'emploi à la composition colorante ou qui est appliquée séquentiellement sans rinçage intermédiaire.

52. Dispositif à plusieurs compartiments ou " Kit " pour la teinture d'oxydation des fibres kératiniques et en particulier des fibres kératiniques humaines telles que les cheveux, **caractérisé par le fait qu'**il comporte au moins deux compartiments, dont l'un d'entre eux renferme une composition (A) telle que définie à l'une quelconque des revendications 1 à 36, et 40 à 49, et un autre, une composition (B) comprenant un agent oxydant et telle que définie à l'une quelconque des revendications 36 à 49.

53. Dispositif à plusieurs compartiments ou " Kit" pour la teinture d'oxydation des fibres kératiniques et en particulier des fibres kératiniques humaines telles que les cheveux, **caractérisé par le fait qu'**il comporte au moins deux compartiments, dont l'un d'entre eux renferme une composition colorante contenant dans un milieu approprié pour la teinture au moins un colorant d'oxydation, au moins un alcool gras mono- ou poly- glycérolé, en la présence ou en l'absence d'un polymère épaississant comportant au moins une chaîne grasse tel que défini à l'une quelconque des revendications 6 à 19, et un autre une composition oxydante contenant un agent oxydant et une quantité efficace d'au moins un polymère épaississant comportant au moins une chaîne grasse tel que défini à l'une quelconque des revendications 6 à 19.

## Claims

1. Composition called composition (A) for the oxidation dyeing of keratinous fibres, in particular human keratinous fibres such as hair, comprising, in an appropriate dyeing medium at least one oxidation dye, **characterized in that** it comprises, in addition, at least one thickening polymer comprising at least one fatty chain and at least one mono- or polyglycerolated fatty alcohol.

2. Composition according to Claim 1, **characterized in that** the mono- or polyglycerolated fatty alcohol has the following structure:
RO- (-CH₂-CH(CH₂OH)-O -) ₙ-H
in which:
R represents a linear or branched, saturated or unsaturated radical comprising from 8 to 40 carbon atoms;
n represents a number between 1 and 30.

3. Composition according to Claim 2, **characterized in that** in the formula, R comprises from 10 to 30 carbon atoms.

4. Composition according to Claim 2, **characterized in that** in the formula, n represents a number between 1 and 10.

5. Composition according to any one of Claims 1 to 4, **characterized in that** the mono- or polyglycerolated fatty alcohol(s) represent, by weight, from 0.01 to 30%, preferably from 0.05 to 20% and more preferably from 0.1 to 15%, of the total weight of the composition.

6. Composition according to any one of Claims 1 to 5, **characterized in that** the thickening polymer comprising at least one fatty chain is anionic, nonionic or cationic.

7. Composition according to Claim 6, **characterized in that** the anionic thickening polymer having a fatty chain comprises at least one hydrophilic unit and at least one allyl ether unit containing a fatty chain.

8. Composition according to Claim 7, **characterized in that** the hydrophilic unit is an ethylenic unsaturated anionic monomer and preferably a vinylcarboxylic acid.

9. Composition according to Claim 7, **characterized in that** the allyl ether unit containing a fatty chain is a monomer having the following formula (I):
CH₂ = C R' CH₂O Bₙ R (I)
in which R' is H or CH₃, B is ethyleneoxy, n is zero or denotes an integer ranging from 1 to 100, R is a hydrocarbon radical chosen from the alkyl, arylalkyl, aryl, alkylaryl or cycloalkyl radicals, comprising 8 to 30 carbon atoms, preferably 10 to 24, and more particularly 12 to 18 carbon atoms.

10. Composition according to Claim 6, **characterized in that** the anionic thickening polymer containing a fatty chain comprises at least one hydrophilic unit of the olefinic unsaturated carboxylic acid type and at least one hydrophobic unit of the (C₁₀-C₃₀) alkyl ester of unsaturated carboxylic acid type.

11. Composition according to Claim 10, **characterized in that** the hydrophilic unit of the olefinic unsaturated carboxylic acid type is a monomer having the following formula (II): 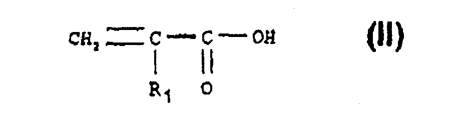 in which R₁ denotes H or CH₃ or C₂H₅, and whose hydrophobic unit of the unsaturated carboxylic acid (C₁₀-C₃₀)alkyl ester of type is a monomer having the following formula (III): 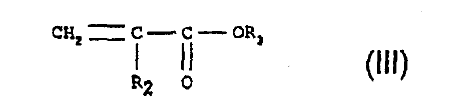 in which R₂ denotes H or CH₃ or C₂H₅, R₃ denoting a C₁₀-C₃₀, and preferably C₁₂-C₂₂, alkyl radical.

12. Composition according to Claim 6, **characterized in that** the anionic thickening polymer containing a fatty chain is a terpolymer of maleic anhydride/C₃₀-C₃₈ α-olefin/alkyl maleate.

13. Composition according to Claim 6, **characterized in that** the anionic thickening polymer containing a fatty chain is an acrylic terpolymer comprising:
(a) a carboxylic acid with α,β-monoethylenic unsaturation
(b) a nonsurfactant monomer with α,β-monoethylenic unsaturation different from (a)
(c) a nonionic monourethane which is the product of the reaction of a monohydric surfactant with a monoisocyanate with monoethylenic unsaturation.

14. Composition according to Claim 6, **characterized in that** the anionic thickening polymer containing a fatty chain is a copolymer of a carboxylic acid with α,β-monoethylenic unsaturation and of an ester of a carboxylic acid with α,β-monoethylenic unsaturation and of oxalkylenated fatty alcohol.

15. Composition according to Claim 6, **characterized in that** the nonionic thickening polymer containing a fatty chain is chosen from the group consisting of:
(1) celluloses modified by groups comprising at least one fatty chain;
(2) hydroxypropylguars modified by groups comprising at least one fatty chain;
(3) polyether-polyurethanes comprising in their chain both hydrophilic sequences of a polyoxyethylenated nature and hydrophobic sequences which are aliphatic chains alone and/or cycloaliphatic and/or aromatic chains;
(4) copolymers of vinylpyrrolidone and of hydrophobic monomers having a fatty chain;
(5) copolymers of C₁-C₆ alkyl methacrylates or acrylates and of amphiphilic monomers comprising at least one fatty chain;
(6) copolymers of hydrophilic methacrylates or acrylates and of hydrophobic monomers comprising at least one fatty chain;
(7) polymers containing an aminoplast ether backbone comprising at least one fatty chain.

16. Composition according to Claim 15, **characterized in that** the polyether-polyurethane comprises at least two lipophilic hydrocarbon chains, having from 6 to 30 carbon atoms, separated by a hydrophilic sequence, the hydrocarbon chains being pendent chains or chains at the end of a hydrophilic sequence.

17. Composition according to Claim 15, **characterized in that** the polyether-polyurethane may be a polyblock sequence, in particular in the form of triblocks.

18. Composition according to Claim 6, **characterized in that** the cationic thickening polymer containing a fatty chain is a quaternized cellulose derivative or a polyacrylate having noncyclic amine-containing side groups.

19. Composition according to any one of Claims 1 to 18, **characterized in that** the thickening polymer(s) containing a fatty chain are present in an amount of 0.01 to 10% and preferably of 0.1 to 5% by weight of the total weight of the composition.

20. Composition according to any one of the preceding claims, **characterized in that** the oxidation dye is chosen from oxidation bases and couplers.

21. Composition according to any one of the preceding claims, **characterized in that** it contains at least one oxidation base.

22. Composition according to Claim 20 or 21, **characterized in that** the oxidation bases are chosen from ortho- or para-phenylenediamines, double bases, ortho- or para-aminophenols and heterocyclic bases, as well as the addition salts of these compounds with an acid.

23. Composition according to Claim 22, **characterized in that** the para-phenylenediamines are chosen from the compounds having the following structure (I): 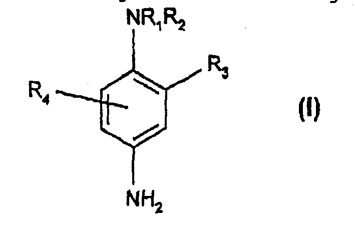 in which:
R₁ represents a hydrogen atom, a C₁-C₄ alkyl radical, a monohydroxy(C₁-C₄ alkyl) radical, a polyhydroxy-(C₂-C₄ alkyl) radical, a (C₁-C₄) alkoxy (C₁-C₄) alkyl radical, a C₁-C₄ alkyl radical substituted with a nitrogen-containing group, a phenyl radical or a 4'-aminophenyl radical;
R₂ represents a hydrogen atom, a C₁-C₄ alkyl radical, a monohydroxy (C₁-C₄ alkyl) radical or a polyhydroxy(C₂-C₄ alkyl) radical, a (C₁-C₄) alkoxy (C₁-C₄) alkyl radical or a C₁-C₄ alkyl radical substituted with a nitrogen-containing group;
R₁ and R₂ may also form, with the nitrogen atom carrying them, a 5- or 6-membered nitrogen-containing heterocycle optionally substituted with one or more alkyl, hydroxyl or ureido groups;
R₃ represents a hydrogen atom, a halogen atom such as a chlorine atom, a C₁-C₄ alkyl radical, a sulpho radical, a carboxyl radical, a monohydroxy(C₁-C₄ alkyl) radical or a hydroxy (C₁-C₄ alkoxy) radical, an acetylamino(C₁-C₄ alkoxy) radical, a mesylamino(C₁-C₄ alkoxy) radical or a carbamoylamino (C₁-C₄ alkoxy) radical,
R₄ represents a hydrogen or halogen atom or a C₁-C₄ alkyl radical.

24. Composition according to Claim 22, **characterized in that** the double bases are chosen from the compounds having the following structure (II): 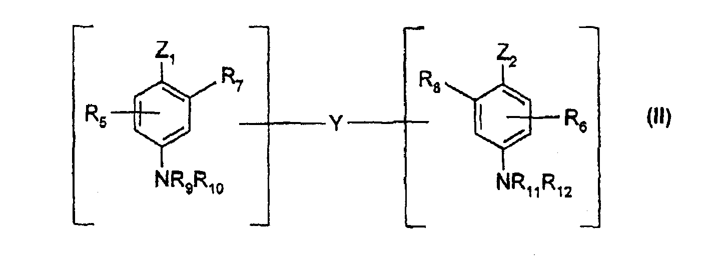 in which:
- Z₁ and Z₂, which are identical or different, represent a hydroxyl or -NH₂ radical which may be substituted with a C₁-C₄ alkyl radical or with a linking arm Y;
- the linking arm Y represents a linear or branched alkylene chain comprising from 1 to 14 carbon atoms, which may be interrupted by or which may end with one or more nitrogen-containing groups and/or one or more heteroatoms such as oxygen, sulphur or nitrogen atoms, and optionally substituted with one or more hydroxyl or C₁-C₆ alkoxy radicals;
- R₅ and R₆ represent a hydrogen or halogen atom, a C₁-C₄ alkyl radical, a monohydroxy(C₁-C₄ alkyl) radical, a polyhydroxy(C₂-C₄ alkyl) radical, an amino(C₁-C₄ alkyl) radical or a linking arm Y;
- R₇, R₈, R₉, R₁₀, R₁₁ and R₁₂, which are identical or different, represent a hydrogen atom, a linking arm Y or a C₁-C₄ alkyl radical;
it being understood that the compounds of formula (II) contain only one linking arm Y per molecule.

25. Composition according to Claim 23 or 24, **characterized in that** the nitrogen-containing groups are chosen from amino, mono (C₁-C₄) alkylamino, (C₁-C₄) dialkylamino, (C₁-C₄) trialkylamino, monohydroxy (C₁-C₄) alkylamino, imidazolinium and ammonium radicals.

26. Composition according to Claim 22, **characterized in that** the para-aminophenols are chosen from the compounds having the following structure (III): 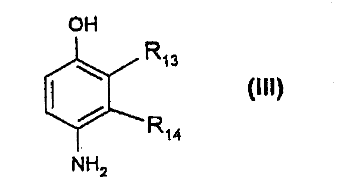 in which:
R₁₃ represents a hydrogen atom, a halogen atom such as fluorine, a C₁-C₄ alkyl, monohydroxy (C₁-C₄ alkyl) , (C₁-C₄) alkoxy (C₁-C₉) alkyl or amino (C₁-C₄ alkyl) or hydroxy (C₁-C₄) alkylamino (C₁-C₄ alkyl) radical,
R₁₄ represents a hydrogen atom or a halogen atom such as fluorine, a C₁-C₄ alkyl, monohydroxy (C₁-C₄ alkyl), polyhydroxy (C₂-C₄ alkyl), amino (C₁-C₄ alkyl), cyano(C₁-C₄ alkyl) or (C₁-C₄)alkoxy(C₁-C₄)alkyl radical.

27. Composition according to Claim 22, **characterized in that** the heterocyclic bases are chosen from pyridine derivatives, pyrimidine derivatives including pyrazolopyrimidines, and pyrazole derivatives.

28. Composition according to Claims 20 to 27, **characterized in that** the oxidation bases are present in concentrations ranging from 0.0005 to 12% by weight relative to the total weight of the composition.

29. Composition according to Claim 20 or 21, **characterized in that** the couplers are chosen from meta-phenylenediamines, meta-aminophenols, meta-diphenols, heterocyclic couplers, and the addition salts of these compounds with an acid.

30. Composition according to Claim 20, 21, or 29, **characterized in that** the couplers are present in concentrations ranging from 0.0001 to 10% by weight relative to the total weight of the composition.

31. Composition according to Claim 22 or 29, **characterized in that** the addition salts with an acid of the oxidation bases and of the couplers are chosen from hydrochlorides, hydrobromides, sulphates, tartrates, lactates and acetates.

32. Composition according to any one of the preceding claims, **characterized in that** it contains, in addition, direct dyes.

33. Composition according to any one of the preceding claims, **characterized in that** it contains, in addition, at least one reducing agent, in quantities ranging from 0.05 to 3% by weight relative to the total weight of the composition.

34. Composition according to any one of the preceding claims, **characterized in that** it contains, in addition, one or more fatty alcohols.

35. Composition according to Claim 34, **characterized in that** the fatty alcohol(s) represent by weight from 0.001 to 20% of the total weight of the composition.

36. Ready-to-use composition for the oxidation dyeing of keratinous fibres, in particular human keratinous fibres such as hair, **characterized in that** it is obtained by mixing a composition (A) as defined in any one of Claims 1 to 35 and a composition (B) containing at least one oxidizing agent.

37. Composition according to Claim 36, **characterized in that** the oxidizing agent is chosen from hydrogen peroxide, urea peroxide, alkali metal bromates or ferricyanides, persalts, oxidation-reduction enzymes such as laccases, peroxidases and oxidoreductases containing 2 electrons, where appropriate in the presence of their respective donor or cofactor.

38. Composition according to Claim 37, **characterized in that** the oxidizing agent is hydrogen peroxide.

39. Composition according to Claim 38, **characterized in that** the oxidizing agent is a solution of hydrogen peroxide whose titre varies from 1 to 40 volumes.

40. Composition according to any one of Claims 1 to 39, **characterized in that** it possesses a pH ranging from 4 to 12.

41. Composition according to Claim 36, **characterized in that** the composition (A) and/or the composition (B) contains at least one cationic or amphoteric polymer.

42. Composition according to Claim 41, **characterized in that** the cationic polymer is a quaternary polyammonium consisting of recurring units corresponding to the following formula (W):

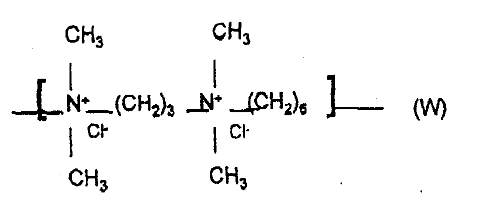

43. Composition according to Claim 41, **characterized in that** the cationic polymer is a quaternary polyammonium consisting of recurring units corresponding to the following formula (U):

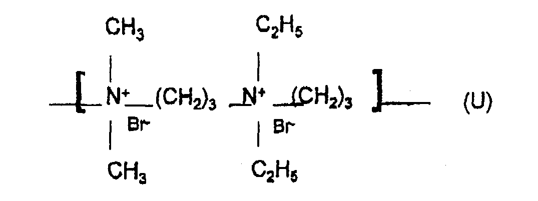

44. Composition according to Claim 41, **characterized in that** the amphoteric polymer is a copolymer comprising at least as monomer acrylic acid and a salt of dimethyldiallylammonium.

45. Composition according to any one of Claims 41 to 44, **characterized in that** the cationic or amphoteric polymer(s) represent from 0.01% to 10%, preferably from 0.05% to 5%, and more preferably from 0.1% to 3% by weight, of the total weight of the composition.

46. Composition according to Claim 36, **characterized in that** the composition (A) and/or the composition (B) contains at least one additional surfactant chosen from anionic, cationic, nonionic or amphoteric surfactants.

47. Composition according to Claim 46, **characterized in that** the additional surfactants represent 0.01 to 40% and preferably 0.1 to 30% of the total weight of the composition.

48. Composition according to Claim 36, **characterized in that** the composition (A) and/or the composition (B) contains a thickening agent chosen from cellulose derivatives, guar derivatives, gums of microbial origin, synthetic thickeners which do not possess a fatty chain.

49. Composition according to Claim 48, **characterized in that** the thickening agent(s) represent from 0.01 to 10% by weight of the total weight of the composition.

50. Method of dyeing keratinous fibres and in particular human keratinous fibres such as hair, **characterized in that** it consists in applying to the fibres a dyeing composition (A) as defined in any one of Claims 1 to 35, and 40 to 49 and in developing the colour in an alkaline, neutral or acidic medium with the aid of a composition (B) containing at least one oxidizing agent, the said composition (B) being defined in any one of Claims 36 to 49 and added just at the time of use to the composition (A) or applied sequentially without intermediate rinsing.

51. Method of dyeing keratinous fibres and in particular human keratinous fibres such as hair, **characterized in that** it consists in applying to the fibres a dyeing composition containing, in an appropriate dyeing medium, at least one oxidation dye, at least one mono- or polyglycerolated fatty alcohol, in the presence or in the absence of a thickening polymer comprising at least one fatty chain as defined in any one of Claims 6 to 19, and in developing the colour in an alkaline, neutral or acidic medium with the aid of an oxidizing composition containing an oxidizing agent and an effective quantity of at least one thickening polymer comprising at least one fatty chain as defined in any one of Claims 6 to 19, and which is mixed just at the time of use with the dyeing composition or which is applied sequentially without intermediate rinsing.

52. Multicompartment device or "kit" for the oxidation dyeing of keratinous fibres and in particular human keratinous fibres such as hair, **characterized in that** it comprises at least two compartments, one of which contains a composition (A) as defined in any one of Claims 1 to 36, and 40 to 49, and another, a composition (B) comprising an oxidizing agent as defined in any one of Claims 36 to 49.

53. Multicompartment device or "kit" for the oxidation dyeing of keratinous fibres and in particular human keratinous fibres such as hair, **characterized in that** it comprises at least two compartments, one of which contains a dyeing composition containing, in an appropriate dyeing medium, at least one oxidation dye, at least one mono- or polyglycerolated fatty alcohol, in the presence or in the absence of a thickening polymer comprising at least one fatty chain as defined in any one of Claims 6 to 19, and another an oxidizing composition containing an oxidizing agent and an effective quantity of at least one thickening polymer comprising at least one fatty chain as defined in any one of Claims 6 to 19.

## Patentansprüche

1. Zusammensetzung, die als Zusammensetzung (A) bezeichnet wird, zum oxidativen Färben von Keratinfasern, insbesondere menschlichen Keratinfasern, wie Haaren, die in einem zum Färben geeigneten Medium mindestens einen Oxidationsfarbstoff enthält, **dadurch gekennzeichnet, dass** sie ferner mindestens ein verdickendes Polymer, das mindestens eine Fettkette aufweist, und mindestens einen ein- oder mehrfach mit Glycerin veretherter Fettalkohol enthält.

2. Zusammensetzung nach Anspruch 1, **dadurch gekennzeichnet, dass** der ein- oder mehrfach mit Glycerin veretherte Fettalkohol die folgende Struktur aufweist:
**RO- (-CH₂-CH(CH₂OH)-O-)ₙ -H**
worin bedeuten:
R eine gesättigte oder ungesättigte, lineare oder verzweigte Gruppe mit 8 bis 40 Kohlenstoffatomen;
n eine Zahl im Bereich von 1 bis 30.

3. Zusammensetzung nach Anspruch 2, **dadurch gekennzeichnet, dass** die Gruppe R in der Formel 10 bis 30 Kohlenstoffatome aufweist.

4. Zusammensetzung nach Anspruch 2, **dadurch gekennzeichnet, dass** n in der Formel eine Zahl von 1 bis 10 bedeutet.

5. Zusammensetzung nach einem der Ansprüche 1 bis 4, **dadurch gekennzeichnet, dass** der oder die ein- oder mehrfach mit Glycerin veretherten Fettalkohole 0,01 bis 30 Gew.-%, vorzugsweise 0,05 bis 20 Gew.-% und noch bevorzugter 0,1 bis 15 Gew.-% des Gesamtgewichts der Zusammensetzung ausmachen.

6. Zusammensetzung nach einem der Ansprüche 1 bis 5, **dadurch gekennzeichnet, dass** das verdickende Polymer, das mindestens eine Fettkette aufweist, anionisch, nichtionisch oder kationisch ist.

7. Zusammensetzung nach Anspruch 6, **dadurch gekennzeichnet, dass** das anionische Polymer mit Fettkette mindestens eine hydrophile Einheit und mindestens eine Allylethereinheit mit Fettkette aufweist.

8. Zusammensetzung nach Anspruch 7, **dadurch gekennzeichnet, dass** die hydrophile Einheit ein ethylenisch ungesättigtes anionisches Monomer und vorzugsweise eine Vinylcarbonsäure ist.

9. Zusammensetzung nach Anspruch 7, **dadurch gekennzeichnet, dass** die Allylethereinheit mit Fettkette ein Monomer der folgenden Formel (I) ist;
**CH₂ = C R' CH₂ O Bₙ R (I)**
worin R' H oder CH₃ bedeutet, B Ethylenoxy ist, n Null ist oder eine ganze Zahl von 1 bis 100 bedeutet, R eine Kohlenwasserstoffgruppe ist, die unter den Gruppen Alkyl, Arylalkyl, Aryl, Alkylaryl und Cycloalkyl mit 8 bis 30 Kohlenstoffatomen, vorzugsweise 10 bis 24 Kohlenstoffatomen und insbesondere 12 bis 18 Kohlenstoffatomen ausgewählt ist.

10. Zusammensetzung nach Anspruch 6, **dadurch gekennzeichnet, dass** das anionische Polymer mit Fettkette mindestens eine hydrophile Einheit vom Typ einer olefinisch ungesättigten Carbonsäure und mindestens eine hydrophobe Einheit vom Typ eines Alkyl(C₁₀₋₃₀)esters einer ungesättigten Carbonsäure aufweist.

11. Zusammensetzung nach Anspruch 10, **dadurch gekennzeichnet, dass** die hydrophile Einheit vom Typ einer olefinisch ungesättigten Carbonsäure einem Monomer der folgenden Formel (II) entspricht: 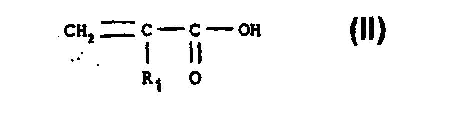 worin die Gruppe R₁ H oder CH₃ oder C₂H₅ bedeutet, und deren hydrophobe Einheit vom Typ eines Alkyl(C₁₀₋₃₀)esters einer ungesättigten Carbonsäure einem Monomer der folgenden Formel (III) entspricht: 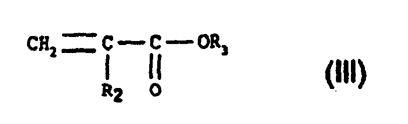 worin die Gruppe R₂ H oder CH₃ oder C₂H₅ bedeutet und R₃ eine C₁₀₋₂₀-Alkylgruppe und vorzugsweise eine C₁₂₋₂₂-Alkylgruppe ist.

12. Zusammensetzung nach Anspruch 6, **dadurch gekennzeichnet, dass** das anionische verdickende Polymer mit Fettkette ein Maleinsäureanhydrid /C₃₀₋₃₈-α-Olefin/Alkylmaleat-Terpolymer ist.

13. Zusammensetzung nach Anspruch 6, **dadurch gekennzeichnet, dass** das anionische verdickende Polymer mit Fettkette ein Acrylterpolymer ist, das enthält:
(a) eine Carbonsäure mit α,β-monoethylenisch ungesättigter Bindung,
(b) ein Monomer mit α,β-monoethylenisch ungesättigter Bindung, das nicht grenzflächenaktiv und von (a) verschieden ist,
(c) ein nichtionisches Monourethan, das das Reaktionsprodukt eines Monowasserstoff-Tensids mit einem Monoisocyanat mit monoethylenisch ungesättigter Bindung ist.

14. Zusammensetzung nach Anspruch 6, **dadurch gekennzeichnet, dass** das anionische verdickende Polymer mit Fettkette ein Copolymer einer Carbonsäure mit α,β-monoethylenisch ungesättigter Bindung und eines Esters einer Carbonsäure mit α, β-monoethylenisch ungesättigter Bindung und eines alkoxylierten Fettalkohols ist.

15. Zusammensetzung nach Anspruch 6, **dadurch gekennzeichnet, dass** das nichtionische verdickende Polymer mit Fettkette ausgewählt ist unter:
(1) Cellulosen, die mit Gruppen modifiziert sind, die mindestens eine Fettkette aufweisen;
(2) Hydroxypropylguargummen, die mit Gruppen modifiziert sind, die mindestens eine Fettkette enthalten;
(3) Polyurethanpolyethern, die in ihrer Kette gleichzeitig hydrophile Sequenzen vom Polyoxyethylentyp und hydrophobe Sequenzen enthalten, bei denen es sich um aliphatische Ketten alleine und/oder cycloaliphatische Ketten und/oder aromatische Ketten handelt;
(4) Copolymeren von Vinylpyrrolidon und hydrophoben Monomeren mit Fettkette;
(5) Copolymeren von C₁-₆-Alkylmethacrylaten oder C₁-₆-Alkylacrylaten und amphiphilen Monomeren, die mindestens eine Fettkette enthalten;
(6) Copolymeren von hydrophilen Methacrylaten oder Acrylaten und hydrophoben Monomeren, die mindestens eine Fettkette enthalten;
(7) Polymeren mit Aminoplastether-Grundgerüst, die mindestens eine Fettkette tragen.

16. Zusammensetzung nach Anspruch 15, **dadurch gekennzeichnet, dass** das Polyetherpolyurethan mindestens zwei lipophile Kohlenwasserstoffketten mit 6 bis 30 Kohlenstoffatomen aufweist, die über eine hydrophile Sequenz getrennt sind, wobei die Kohlenwasserstoffketten Seitenketten oder Ketten am Ende der hydrophilen Sequenz sind.

17. Zusammensetzung nach Anspruch 15, **dadurch gekennzeichnet, dass** das Polyetherpolyurethan multisequentiell insbesondere in Triblockform vorliegen kann.

18. Zusammensetzung nach Anspruch 6, **dadurch gekennzeichnet, dass** das kationische verdickende Polymer mit Fettkette ein quaternisiertes Cellulosederivat oder ein Polyacrylat mit nicht cyclischen, aminierten Seitengruppen ist.

19. Zusammensetzung nach einem der Ansprüche 1 bis 18, **dadurch gekennzeichnet, dass** das oder die verdickenden Polymere mit Fettkette in einer Menge von 0,01 bis 10 % und vorzugsweise 0,1 bis 5 Gew.-% des Gesamtgewichts der Zusammensetzung enthalten sind.

20. Zusammensetzung nach einem der vorhergehenden Ansprüche, **dadurch gekennzeichnet, dass** der Oxidationsfarbstoff unter den Oxidationsbasen und Kupplern ausgewählt ist.

21. Zusammensetzung nach einem der vorhergehenden Ansprüche, **dadurch gekennzeichnet, dass** die Zusammensetzung mindestens eine Oxidationsbase enthält.

22. Zusammensetzung nach den Ansprüchen 20 oder 21, **dadurch gekennzeichnet, dass** die Oxidationsbasen unter den *o*- oder *p*-Phenylendiaminen, Doppelbasen, *o*- oder *p*-Aminophenolen und den heterocyclischen Basen sowie den Additionssalzen dieser Verbindungen mit einer Säure ausgewählt sind.

23. Zusammensetzung nach Anspruch 22, **dadurch gekennzeichnet, dass** die *p*-Phenylendiamine unter den Verbindungen der folgenden Formel (I) ausgewählt sind: 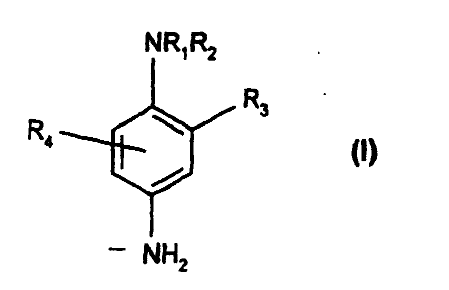 worin bedeuten:
R₁ ein Wasserstoffatom, C₁₋₄-Alkyl, C₁₋₄-Monohydroxyalkyl, C₂₋₄-Polyhydroxyalkyl, Alkoxy(C₁₋₄)alkyl(C₁₋₄), eine mit einer Stickstoff-haltigen Gruppe substituierte C₁₋₄-Alkylgruppe, Phenyl oder 4'-Aminophenyl;
R₂ ein Wasserstoffatom, C₁₋₄-Alkyl, C₁₋₄-Monohydroxyalkyl, C₂₋₄-Polyhydroxyalkyl, Alkoxy(C₁₋₄)alkyl(C₁₋₄) oder eine mit einer Stickstoff-haltigen Gruppe substituierte C₁₋₄-Alkylgruppe, wobei R₁ und R₂ auch mit dem Stickstoffatom, von dem sie getragen werden, einen 5- oder 6-gliedrigen Stickstoffheterocyclus bilden können, der gegebenenfalls mit einer oder mehreren Gruppen Alkyl, Hydroxy oder Ureido substituiert ist;
R₃ ein Wasserstoffatom, ein Halogenatom, wie Chlor, C₁₋₄-Alkyl, Sulfo, Carboxy, C₁₋₄-Monohydroxyalkyl, C₁₋₄-Hydroxyalkoxy, Acetylaminoalkoxy(C₁₋₄), Mesylaminoalkoxy(C₁₋₄) oder Carbamoyl-aminoalkoxy(C₁₋₄),
R₄ ein Wasserstoffatom, ein Halogenatom oder C₁₋₄-Alkyl.

24. Zusammensetzung nach Anspruch 22, **dadurch gekennzeichnet, dass** die Doppelbasen unter den Verbindungen der folgenden Struktur (II) ausgewählt sind: 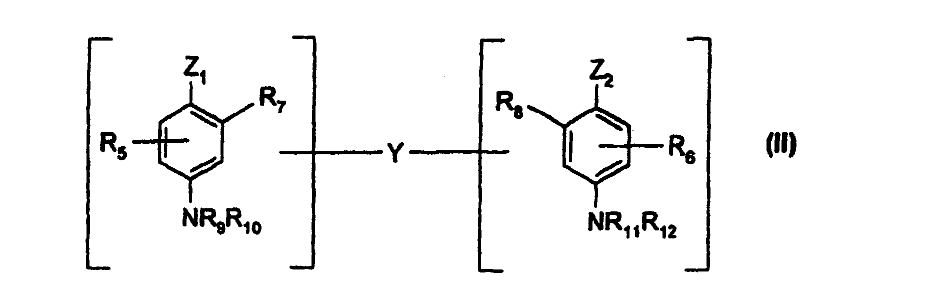 worin bedeuten:
- die Gruppen Z₁ und Z₂, die gleich oder verschieden sind, die Hydroxygruppe oder die Gruppe -NH₂, die mit einer C₁₋₄-Alkylgruppe oder einer Verbindungsgruppe Y substituiert sein können;
- die Verbindungsgruppe Y eine geradkettige oder verzweigte Alkylenkette mit 1 bis 14 Kohlenstoffatomen, die durch eine oder mehrere Stickstoff-haltige Gruppen und/oder ein oder mehrere Heteroatome, wie Sauerstoff, Schwefel oder Stickstoff, unterbrochen oder abgeschlossen sein kann und gegebenenfalls mit einer oder mehreren Hydroxygruppen oder C₁₋₆-Alkoxygruppen substituiert ist;
- die Gruppen R₅ und R₆ ein Wasserstoffatom, ein Halogenatom, C₁₋₄-Alkyl, C₁₋₄-Monohydroxyalkyl, C₂₋₄-Polyhydroxyalkyl, C₁₋₄-Aminoalkyl oder eine Verbindungsgruppe Y;
- die Gruppen R₇, R₈, R₉, R₁₀, R₁₁ und R₁₂, die gleich oder verschieden sind, ein Wasserstoffatom, eine Verbindungsgruppe Y oder eine C₁-₄-Alkylgruppe;
- mit der Maßgabe, dass die Verbindungen der Formel (II) nur eine Verbindungsgruppe Y pro Molekül enthalten.

25. Zusammensetzung nach den Ansprüchen 23 oder 24, **dadurch gekennzeichnet, dass** die Stickstoff-haltigen Gruppen unter den Gruppen Amino, Monoalkyl(C₁-₄)amino, Dialkyl(C₁-₄)amino, Trialkyl(C₁-₄)amino, Monohydroxyalkyl(C₁-₄)amino, Imidazolinium und Ammonium ausgewählt sind.

26. Zusammensetzung nach Anspruch 22, **dadurch gekennzeichnet, dass** die *p*-Aminophenole unter den Verbindungen der folgenden Struktur (III) ausgewählt sind: 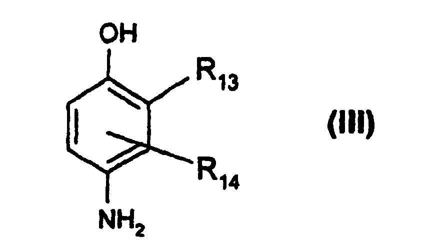 worin bedeuten:
- R₁₃ ein Wasserstoffatom, ein Halogenatom, wie Fluor, C₁₋₄-Alkyl, C₁₋₄-Monohydroxyalkyl, Alkoxy(C₁₋₄)alkyl(C₁₋₄), C₁₋₄-Aminoalkyl oder Hydroxyalkyl(C₁₋₄)aminoalkyl(C₁₋₄),
- R₁₄ ein Wasserstoffatom, ein Halogenatom, wie Fluor, C₁₋₄-Alkyl, C₁₋₄-Monohydroxyalkyl, C₂₋₄-Polyhydroxyalkyl, C₁₋₄-Aminoalkyl, Cyanoalkyl(C₁₋₄) oder Alkoxy(C₁₋₄)alkyl(C₁₋₄).

27. Zusammensetzung nach Anspruch 22, **dadurch gekennzeichnet, dass** die heterocyclischen Basen unter den Pyridinderivaten, Pyrimidinderivaten und darunter Pyrazolopyrimidinen und Pyrazolderivaten ausgewählt sind.

28. Zusammensetzung nach den Ansprüchen 20 bis 27, **dadurch gekennzeichnet, dass** die Oxidationsbasen in Konzentrationen von 0,0005 bis 12 Gew.-%, bezogen auf das Gesamtgewicht der Zusammensetzung, enthalten sind.

29. Zusammensetzung nach den Ansprüchen 20 oder 21, **dadurch gekennzeichnet, dass** die Kuppler unter den *m*-Phenylendiaminen, *m*-Aminophenolen, *m*-Dihydroxybenzolen, heterocyclischen Kupplern und den Additionssalzen dieser Verbindungen mit einer Säure ausgewählt sind.

30. Zusammensetzung nach den Ansprüchen 20, 21 oder 29, **dadurch gekennzeichnet, dass** die Kuppler in Konzentrationen von 0,0001 bis 10 Gew.-%, bezogen auf das Gesamtgewicht der Zusammensetzung, enthalten sind.

31. Zusammensetzung nach den Ansprüchen 22 oder 29, **dadurch gekennzeichnet, dass** die Additionssalze der Oxidationsbasen und Kuppler mit einer Säure unter den Hydrochloriden, Hydrobromiden, Sulfaten, Tartraten, Lactaten und Acetaten ausgewählt sind.

32. Zusammensetzung nach einem der vorhergehenden Ansprüche, **dadurch gekennzeichnet, dass** die Zusammensetzung ferner Direktfarbstoffe enthält.

33. Zusammensetzung nach einem der vorhergehenden Ansprüche, **dadurch gekennzeichnet, dass** die Zusammensetzung ferner mindestens ein Reduktionsmittel in Mengenanteilen von 0,05 bis 3 Gew.-%, bezogen auf das Gesamtgewicht der Zusammensetzung enthält.

34. Zusammensetzung nach einem der vorhergehenden Ansprüche, **dadurch gekennzeichnet, dass** sie ferner einen oder mehrere Fettalkohole enthält.

35. Zusammensetzung nach Anspruch 34, **dadurch gekennzeichnet, dass** der oder die Fettalkohole 0,001 bis 20 Gew.-% des Gesamtgewichts der Zusammensetzung ausmachen.

36. Gebrauchsfertige Zusammensetzung zum oxidativen Färben von Keratinfasern und insbesondere menschlichen Keratinfasern, wie Haaren, **dadurch gekennzeichnet, dass** sie durch Mischen einer Zusammensetzung (A), wie sie in den Ansprüchen 1 bis 35 definiert ist, und einer Zusammensetzung (B) gebildet wird, die mindestens ein Oxidationsmittel enthält.

37. Zusammensetzung nach Anspruch 36, **dadurch gekennzeichnet, dass** das Oxidationsmittel unter Wasserstoffperoxid, Harnstoff peroxid, Alkalimetallbromaten oder -ferricyaniden, Salzen von Persäuren, Redoxenzymen, wie Laccasen, Peroxidasen und Oxidoreduktasen (2 Elektronen) gegebenenfalls in Gegenwart ihres jeweiligen Donors oder Cofaktors ausgewählt ist.

38. Zusammensetzung nach Anspruch 37, **dadurch gekennzeichnet, dass** es sich bei dem Oxidationsmittel um Wasserstoffperoxid handelt.

39. Zusammensetzung nach Anspruch 38, **dadurch gekennzeichnet, dass** das Oxidationsmittel eine Wasserstoffperoxidlösung ist, deren Titer im Bereich von 1 bis 40 Volumina liegt.

40. Zusammensetzung nach einem der Ansprüche 1 bis 39, **dadurch gekennzeichnet, dass** sie einen pH-Wert von 4 bis 12 aufweist.

41. Zusammensetzung nach Anspruch 36, **dadurch gekennzeichnet, dass** die Zusammensetzung (A) und/ oder die Zusammensetzung (B) mindestens ein kationisches oder amphoteres Polymer enthält.

42. Zusammensetzung nach Anspruch 41, **dadurch gekennzeichnet, dass** das kationische Polymer ein quartäres Polyammoniumpolymer ist, das aus wiederkehrenden Einheiten der folgenden Formel (W) besteht:

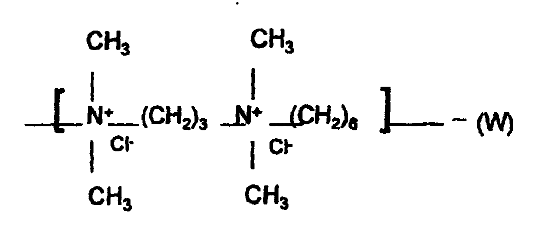

43. Zusammensetzung nach Anspruch 41, **dadurch gekennzeichnet, dass** das kationische Polymer ein quartäres Polyammoniumpolymer ist, das aus wiederkehrenden Einheiten der folgenden Formel (U) besteht:

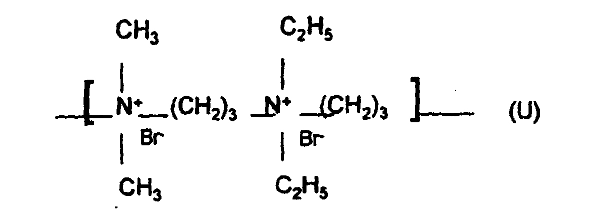

44. Zusammensetzung nach Anspruch 41, **dadurch gekennzeichnet, dass** das amphotere Polymer ein Copolymer ist, das als Monomer zumindest die Acrylsäure und ein Dimethyldiallylammoniumsalz enthält.

45. Zusammensetzung nach einem der Ansprüche 41 bis 44, **dadurch gekennzeichnet, dass** das oder die kationische(n) oder amphotere(n) Polymer(e) 0,01 bis 10 Gew.-%, vorzugsweise 0,05 bis 5 Gew.-% und noch bevorzugter 0,1 bis 3 Gew.-% des Gesamtgewichts der Zusammensetzung ausmachen.

46. Zusammensetzung nach Anspruch 36, **dadurch gekennzeichnet, dass** die Zusammensetzung (A) und/ oder die Zusammensetzung (B) mindestens einen ergänzenden grenzflächenaktiven Stoff enthält, der unter den anionischen, kationischen, nichtionischen oder amphoteren grenzflächenaktiven Stoffen ausgewählt ist.

47. Zusammensetzung nach Anspruch 46, **dadurch gekennzeichnet, dass** die grenzflächenaktiven Stoffe 0,01 bis 40 % und vorzugsweise 0,1 bis 30 % des Gesamtgewichts der Zusammensetzung, ausmachen.

48. Zusammensetzung nach Anspruch 36, **dadurch gekennzeichnet, dass** die Zusammensetzung (A) und/ oder die Zusammensetzung (B) ein Verdickungsmittel enthält, das unter den Cellulosederivaten, Guarderivaten, Gummen mikrobieller Herkunft, synthetischen Verdickungsmitteln, die keine Fettkette aufweisen, ausgewählt ist.

49. Zusammensetzung nach Anspruch 48, **dadurch gekennzeichnet, dass** das oder die Verdickungsmittel 0,01 bis 10 Gew.-% des Gesamtgewichts der Zusammensetzung ausmachen.

50. Verfahren zum Färben von Keratinfasern und insbesondere menschlichen Keratinfasern, wie Haaren, **dadurch gekennzeichnet, dass** es darin besteht, auf die Fasern eine Farbmittelzusammensetzung (A), wie sie in einem der Ansprüche 1 bis 35 und 40 bis 49 definiert ist, aufzutragen und die Farbe in einem alkalischen, neutralen oder sauren Medium mit einer Zusammensetzung (B) zu bilden, die mindestens ein Oxidationsmittel enthält,
wobei die Zusammensetzung (B), die in einem der Ansprüche 36 bis 49 definiert wurde, bei der Anwendung zu der Zusammensetzung (A) gegeben wird oder ohne zwischenzeitliches Spülen getrennt davon anschließend aufgetragen wird.

51. Verfahren zum Färben von Keratinfasern und insbesondere menschlichen Keratinfasern, wie Haaren, **dadurch gekennzeichnet, dass** es darin besteht, auf die Fasern eine Farbmittelzusammensetzung aufzutragen, die in einem zum Färben geeigneten Medium mindestens einen Oxidationsfarbstoff und mindestens einen ein- oder mehrfach mit Glycerin veretherten Fettalkohol in Gegenwart oder in Abwesenheit eines verdickenden Polymers, das mindestens eine Fettkette aufweist, nach einem der Ansprüche 6 bis 19 enthält, und die Farbe in einem alkalischen, neutralen oder sauren Medium mit einer oxidierenden Zusammensetzung zu bilden, die ein Oxidationsmittel und in einer wirksamen Menge mindestens ein verdickendes Polymer, das mindestens eine Fettkette aufweist, nach einem der Ansprüche 6 bis 19 enthält und bei der Anwendung mit der Farbmittelzusammensetzung vermischt wird oder ohne zwischenzeitliches Spülen getrennt davon anschließend aufgetragen wird.

52. Vorrichtung mit mehreren Abteilungen oder "Kit" zum oxidativen Färben von Keratinfasern und insbesondere menschlichen Keratinfasern, wie Haaren, **dadurch gekennzeichnet, dass** mindestens zwei Abteilungen enthalten sind, wobei eine Abteilung eine Zusammensetzung (A) nach einem der Ansprüche 1 bis 36 und 40 bis 49 und eine andere Abteilung eine Zusammensetzung (B) enthält, die ein Oxidationsmittel enthält, nach einem der Ansprüche 36 bis 49.

53. Vorrichtung mit mehreren Abteilungen oder "Kit" zum oxidativen Färben von Keratinfasern und insbesondere menschlichen Keratinfasern, wie Haaren, **dadurch gekennzeichnet, dass** mindestens zwei Abteilungen enthalten sind, wobei eine Abteilung eine Farbmittelzusammensetzung, die in einem zum Färben geeigneten Medium mindestens einen Oxidationsfarbstoff und mindestens einen ein- oder mehrfach mit Glycerin veretherten Fettalkohol in Gegenwart oder in Abwesenheit eines verdickenden Polymers mit mindestens einer Fettkette nach einem der Ansprüche 6 bis 19 enthält, und eine andere Abteilung eine oxidierende Zusammensetzung enthält, die ein Oxidationsmittel und in einer wirksamen Menge mindestens ein verdickendes Polymer mit mindestens einer Fettkette nach einem der Ansprüche 6 bis 19 enthält.
